# EUROPEAN PATENT SPECIFICATION

(11) **EP 4 188 191 B1**
(45) Date of publication and mention of the grant of the patent: **27.05.2026**
(21) Application number: 21746901.4
(22) Date of filing: 09.07.2021
(51) Int. Cl.: A61B 5/00, A61B 5/282, A61B 5/346, A61B 5/29, A61B 5/367, A61B 7/04

(54) **STABLE CARDIAC SIGNAL IDENTIFICATION**
STABILE HERZSIGNALIDENTIFIZIERUNG
IDENTIFICATION DE SIGNAUX CARDIAQUES STABLES

(30) Priority: 31.07.2020 US 202063059473 P; 30.06.2021 US 202117363318
(43) Date of publication of application: 07.06.2023
(73) Proprietor: Medtronic, Inc., Minneapolis, Minnesota 55432 (US)
(72) Inventor: KUDLIK, D'Anne E., Minneapolis, Minnesota 55432 (US); YOON, Sarah, Minneapolis, Minnesota 55432 (US)
(74) Representative: Zimmermann & Partner Patentanwälte mbB
(86) International application number: PCT/US2021/040992
(87) International publication number: WO 2022/026154

(56) References cited:
- US-A1- 2013 304 407
- US-A1- 2014 323 892
- US-A1- 2019 366 106

## Description

The disclosure herein relates to systems and methods for use in identifying stable and unstable cardiac signals monitored from a patient using a plurality of electrodes.

Systems for evaluating the cardia health of a patient and for implanting medical devices may include workstations or other equipment in addition to the implantable medical device itself. In some cases, these other pieces of equipment assist the physician or other technician with placing the intracardiac leads at particular locations on or in the heart. In some cases, the equipment provides information to the physician about the electrical activity of the heart as well as the location of the intracardiac lead, if the [patient is receiving therapy.

In some cases, the workstations or other equipment may include apparatus for obtaining electrocardiograms (ECG) via electrodes on the surface, or skin, of the patient. More specifically, the patient may have a plurality of electrodes on an ECG belt or vest that surrounds the torso of the patient. After the belt or vest has been secured to the torso, a physician can perform a series of tests to evaluate a patient's cardiac response. The data provided by the ECG electrodes placed on the body surface of the patient may be used for various evaluation purposes including determining the cardiac condition of a patient, determining whether a patient would benefit from cardiac therapy, etc. and may be used further therapeutic purposes (e.g., cardiac resynchronization therapy) including optimizing lead location, pacing parameters, etc. based on one or more metrics derived from the signals captured by the ECG electrodes. For example, electrical heterogeneity information (EHI) may be derived from electrical activation times computed from multiple electrodes on the body surface, and the EHI may be used to optimize lead location, pacing parameters, determine whether a patient qualifies for cardiac therapy, determine whether cardiac therapy would likely be effective for a patient, etc.

The cardiac, or electrode, signals monitored from a patient using a plurality of external electrodes attached to the patient's skin may include stable signals suitable for further use and may also include unstable signals that not suitable for further use.

US2019/366106 A1 relates to systems, methods, and interfaces for use in cardiac evaluation. US2014/323892 A1 relates to systems, methods, and interfaces for identifying effective electrodes.

### SUMMARY

The illustrative systems and methods described herein may be configured to determine which cardiac signals are stable and which cardiac signals are unstable so as to be able to determine which cardiac signals may be further utilized. After the stable signals have been identified, the stable cardiac signals can be further utilized to process the cardiac signal data such as identifying QRS complexes (e.g., identifying QRS onsets and offsets). Once the cardiac signal data has been appropriately processed, the illustrative systems and methods may assist a user in evaluating and analyzing a patient's cardiac condition so as to, e.g., determine whether the patient qualifies or would benefit from cardiac therapy, determine whether presently-delivered cardiac therapy is effective, identify the nature of a patient's cardiac condition, etc. In one or more embodiments, the systems and methods may be described as being noninvasive. For example, in some embodiments, the systems and methods may not need, or include, implantable devices such as leads, probes, sensors, catheters, implantable electrodes, etc. to monitor, or acquire, a plurality of cardiac signals from tissue of the patient. Instead, the systems and methods may use electrical measurements taken noninvasively using, e.g., a plurality of external electrodes attached to tissue (e.g., the skin) of a patient about the patient's torso.

One illustrative system may include electrode apparatus comprising a plurality of electrodes to monitor electrical activity from tissue of a patient and computing apparatus comprising processing circuitry and coupled to the electrode apparatus. The computing apparatus may be configured to monitor electrical activity using the plurality of electrodes to generate a plurality of cardiac signals over an analysis time period and generate a dispersion signal from the plurality of cardiac signals. The dispersion signal is representative of the dispersion of the plurality of cardiac signals over the analysis time period. The computing apparatus may be further configured to select a low dispersion time period within the analysis time period based on rate of change of the dispersion signal and determine whether each of the plurality of cardiac signals is stable based on the cardiac signal within the low dispersion time period.

One illustrative method, which is not claimed as such but is useful for understanding the invention, may include monitoring electrical activity from
tissue of a patient using a plurality of electrodes to generate a plurality of cardiac signals over an analysis time period and generating a dispersion signal from the plurality of cardiac signals, where the dispersion signal is representative of the dispersion of the plurality of cardiac signals over the analysis time period. The illustrative method may further include selecting a low dispersion time period within the analysis time period based on rate of change of the dispersion signal and determining whether each of the plurality of cardiac signals is stable based on the cardiac signal within the low dispersion time period.

One illustrative system may include electrode apparatus comprising a plurality of electrodes to monitor electrical activity from tissue of a patient and computing apparatus comprising processing circuitry and coupled to the electrode apparatus. The computing apparatus may be configured to: monitor electrical activity using the plurality of electrodes to generate a plurality of cardiac signals over an analysis time period, select a low dispersion time period within the analysis time period representative of a period of low dispersion of the plurality of cardiac signals, determine whether each of the plurality of cardiac signals is unstable based on each cardiac signal within the low dispersion time period, and remove the cardiac signals determined to be unstable.

The above summary is not intended to describe each embodiment or every implementation of the present disclosure. A more complete understanding will become apparent and appreciated by referring to the following detailed description and claims taken in conjunction with the accompanying drawings.
The invention is defined by the appended claims.

### BRIEF DESCRIPTION OF THE DRAWINGS

FIG. 1 is a diagram of an illustrative system including electrode apparatus, display apparatus, and computing apparatus.
FIGS. 2-3 are diagrams of illustrative external electrode apparatus for measuring torso-surface potentials.
FIG. 4 is a block diagram of an illustrative method of identifying stable cardiac signals.
FIG. 5 is a block diagram of illustrative method of selecting a low dispersion time period.
FIG. 6 is a block diagram of illustrative method of determining which cardiac signals are stable and which cardiac signals are unstable.
FIG. 7A is a graph of a plurality of illustrative cardiac signals.
FIG. 7B is a graph of the peak-to-peak amplitudes of the plurality of cardiac signals of FIG. 7A.
FIG. 7C is a graph of a dispersion signal of the plurality of cardiac signals of FIG. 7A.
FIG. 7D is a graph of a slope of the dispersion signal of FIG. 7C.
FIG. 7E is a graph of a summation over a sliding window of the slope of the dispersion signal of FIG. 7D.
FIG. 7F is a graph of the plurality of illustrative cardiac signals with an initial low dispersion time period identified using the summation over a sliding window of the slope of the dispersion signal of FIG. 7E.
FIG. 7G is a graph of a summation over a sliding window of the slope of the dispersion signal within the low dispersion time period of FIG. 7F.
FIG. 7H is a graph of the plurality of illustrative cardiac signals with the low dispersion time period identified using the summation over a sliding window of the slope of the dispersion signal of FIG. 7G.
FIG. 7I is a graph of the peak-to-peak amplitudes of the plurality of cardiac signals within the low dispersion time period of FIG. 7H.
FIG. 7J a graph of the stable cardiac signals of the plurality of cardiac signals of FIG. 7A.
FIG. 7K a graph of the unstable cardiac signals of the plurality of cardiac signals of FIG. 7A.
FIG. 8 is a diagram of an illustrative system including an illustrative implantable medical device (IMD).
FIG. 9A is a diagram of the illustrative IMD of FIG. 8.
FIG. 9B is a diagram of an enlarged view of a distal end of the electrical lead disposed in the left ventricle of FIG. 9A.
FIG. 10A is a block diagram of an illustrative IMD, e.g., of the systems of FIGS. 8-9.
FIG. 10B is another block diagram of an illustrative IMD (e.g., an implantable pulse generator) circuitry and associated leads employed in the systems of FIGS. 8-9.

### DETAILED DESCRIPTION

In the following detailed description, reference is made to the accompanying figures of the drawing which form a part hereof, and in which are shown, by way of illustration, specific embodiments which may be practiced. It is to be understood that other embodiments may be utilized and structural changes may be made without departing from (e.g., still falling within) the scope of the disclosure presented hereby.

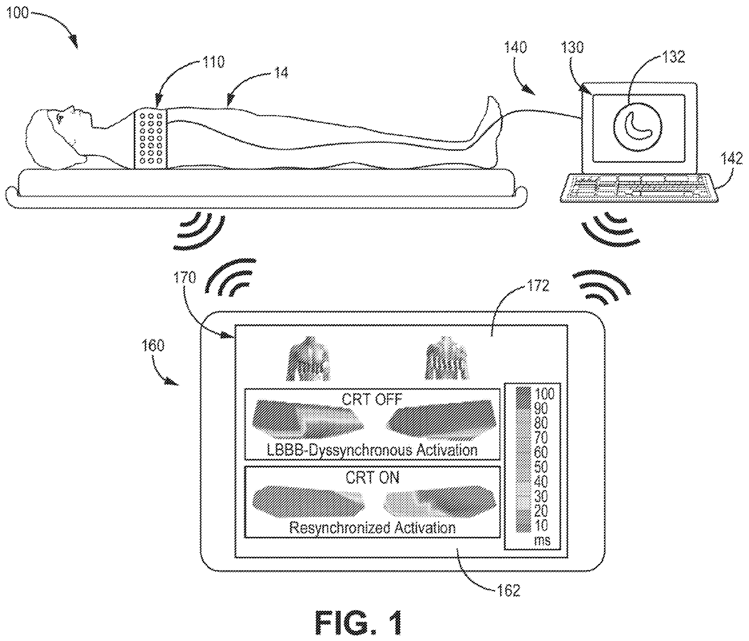

Illustrative systems and methods shall be described with reference to Figures 1-10. It will be apparent to one skilled in the art that elements or processes from one embodiment may be used in combination with elements or processes of the other embodiments, and that the possible embodiments of such systems and methods using combinations of features set forth herein is not limited to the specific embodiments shown in the Figures and/or described herein. Further, it will be recognized that the embodiments described herein may include many elements that are not necessarily shown to scale. Still further, it will be recognized that timing of the processes and the size and shape of various elements herein may be modified but still fall within the scope of the present disclosure, although certain timings, one or more shapes and/or sizes, or types of elements, may be advantageous over others.

A plurality of electrocardiogram (ECG) cardiac signals (e.g., torso-surface potentials) may be measured, or monitored, using a plurality of external electrodes positioned about the surface, or skin, of a patient. The ECG cardiac signals may be used to evaluate a patient's cardiac condition to, e.g., determine whether or not the patient may benefit from cardiac therapy (e.g., cardiac therapy provided by an implantable medical device performing cardiac resynchronization therapy (CRT)). Further, the ECG cardiac signals may be used to implant or configure cardiac therapy. As described herein, the ECG cardiac signals may be gathered or obtained noninvasively since, e.g., implantable electrodes may not be used to measure the ECG cardiac signals. Further, the ECG cardiac signals may be used to determine cardiac electrical activation times, which may be used to generate various metrics (e.g., electrical heterogeneity information). Such various metrics may then be used to determine the patient's cardiac condition, which may then be used to alert the patient to their cardiac condition, to provide an indication that the patient may benefit from cardiac therapy, to provide additional information to a remote care team, to be used to tune or configure cardiac therapy, etc.

Various illustrative systems, methods, devices, and graphical user interfaces may be configured to use electrode apparatus including external electrodes, display apparatus, and computing apparatus to noninvasively assist a user (e.g., a physician) in the evaluation of cardiac health, determination of the benefit of cardiac therapy, and implantation/configuration of cardiac therapy. An illustrative system 100 including electrode apparatus 110, remote computing apparatus 140, and a local computing device 160 is depicted in FIG. 1.

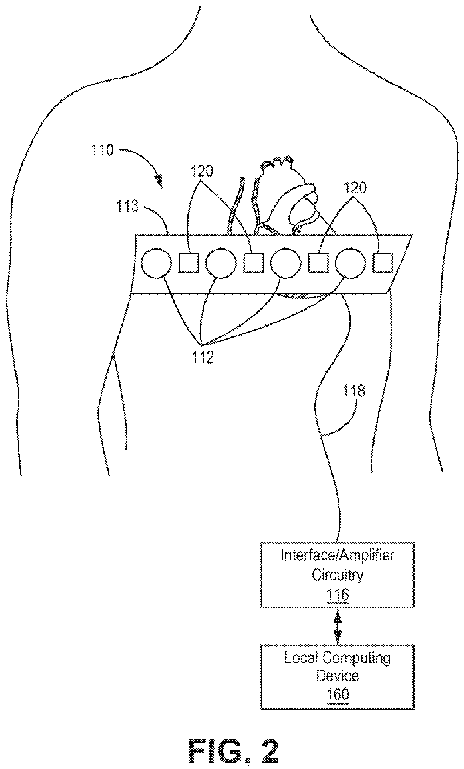

The electrode apparatus 110 as shown includes a plurality of electrodes incorporated, or included, within a band wrapped around the chest, or torso, of a patient 14. The electrode apparatus 110 may be operatively coupled to the local computing device 160 (e.g., through one or wired electrical connections, wirelessly, etc.) to provide cardiac electrical signals from each of the electrodes to the local computing apparatus 160 for analysis, evaluation, etc. Illustrative electrode apparatus may be described in U.S. Patent No. 9,320,446 entitled "Bioelectric Sensor Device and Methods" filed March 27, 2014 and issued on March 26, 2016. Further, illustrative electrode apparatus 110 will be described in more detail in reference to FIGS. 2-3.

Although not described herein, the illustrative system 100 may further include imaging apparatus. The imaging apparatus may be any type of imaging apparatus configured to image, or provide images of, at least a portion of the patient in a noninvasive manner. For example, the imaging apparatus may not use any components or parts that may be located within the patient to provide images of the patient except noninvasive tools such as contrast solution. It is to be understood that the illustrative systems, methods, and interfaces described herein may further use imaging apparatus to provide noninvasive assistance to a user (e.g., a physician) to locate, or place, one or more pacing electrodes proximate the patient's heart in conjunction with the configuration of cardiac therapy.

Systems that may be used in conjunction with the illustrative systems, methods, and devices described herein are described in U.S. Pat. App. Pub. No. 2005/0008210 to Evron et al. published on January 13, 2005, U.S. Pat. App. Pub. No. 2006/0074285 to Zarkh et al. published on April 6, 2006, U.S. Pat. No. 8,731,642 to Zarkh et al. issued on May 20, 2014, U.S. Pat. No. 8,861,830 to Brada et al. issued on October 14, 2014, U.S. Pat. No. 6,980,675 to Evron et al. issued on December 27, 2005, U.S. Pat. No. 7,286,866 to Okerlund et al. issued on October 23, 2007, U.S. Pat. No. 7,308,297 to Reddy et al. issued on December 11, 2011, U.S. Pat. No. 7,308,299 to Burrell et al. issued on December 11, 2011, U.S. Pat. No. 7,321,677 to Evron et al. issued on January 22, 2008, U.S. Pat. No. 7,346,381 to Okerlund et al. issued on March 18, 2008, U.S. Pat. No. 7,454,248 to Burrell et al. issued on November 18, 2008, U.S. Pat. No. 7,499,743 to Vass et al. issued on March 3, 2009, U.S. Pat. No. 7,565,190 to Okerlund et al. issued on July 21, 2009, U.S. Pat. No. 7,587,074 to Zarkh et al. issued on September 8, 2009, U.S. Pat. No. 7,599,730 to Hunter et al. issued on October 6, 2009, U.S. Pat. No. 7,613,500 to Vass et al. issued on November 3, 2009, U.S. Pat. No. 7,742,629 to Zarkh et al. issued on June 22, 2010, U.S. Pat. No. 7,747,047 to Okerlund et al. issued on June 29, 2010, U.S. Pat. No. 7,778,685 to Evron et al. issued on August 17, 2010, U.S. Pat. No. 7,778,686 to Vass et al. issued on August 17, 2010, U.S. Pat. No. 7,813,785 to Okerlund et al. issued on October 12, 2010, U.S. Pat. No. 7,996,063 to Vass et al. issued on August 9, 2011, U.S. Pat. No. 8,060,185 to Hunter et al. issued on November 15, 2011, and U.S. Pat. No. 8,401,616 to Verard et al. issued on March 19, 2013.

The remote computing apparatus 140 and the local computing device 160 may each include display apparatus 130, 160, respectively, that may be configured to display and analyze data such as, e.g., electrical cardiac signals (e.g., electrocardiogram data), electrical activation times, electrical heterogeneity information, indications regarding the patient's cardiac condition, determinations of whether the patient may benefit from cardiac therapy, etc. For example, one cardiac cycle, or one heartbeat, of a plurality of cardiac cycles, or heartbeats, represented by the electrical signals collected or monitored by the electrode apparatus 110 may be analyzed and evaluated by one or both of the remote computing apparatus 140 and the local computing device 160 for one or more metrics including activation times and electrical heterogeneity information that may be pertinent to the determination of the patient's cardiac condition and indication of cardiac therapy benefit. More specifically, for example, the QRS complex of a single cardiac cycle may be evaluated for one or more metrics such as, e.g., QRS onset, QRS offset, QRS peak, electrical activation times referenced to the earliest activation time, electrical heterogeneity information (EHI) such as left ventricular or thoracic standard deviation of electrical activation times (LVED), standard deviation of activation times (SDAT), average left ventricular or thoracic surrogate electrical activation times (LVAT), QRS duration (e.g., interval between QRS onset to QRS offset), differences between average left surrogate and average right surrogate activation times, relative or absolute QRS morphology, difference between a higher percentile and a lower percentile of activation times (higher percentile may be 90%, 80%, 75%, 70%, etc. and lower percentile may be 10%, 15%, 20%, 25% and 30%, etc.), other statistical measures of central tendency (e.g., median or mode), dispersion (e.g., mean deviation, standard deviation, variance, interquartile deviations, range), etc. Further, each of the one or more metrics may be location specific. For example, some metrics may be computed from signals recorded, or monitored, from electrodes positioned about a selected area of the patient such as, e.g., the left side of the patient, the right side of the patient, etc.

In at least one embodiment, one or both of the remote computing apparatus 140 and the local computing device 160 may be a server, a personal computer, smartphone, or a tablet computer. The remote computing apparatus 140 may be configured to receive input from input apparatus 142 (e.g., a keyboard) and transmit output to the display apparatus 130, and the local computing device 160 may be configured to receive input from input apparatus 162 (e.g., a touchscreen) and transmit output to the display apparatus 170. One or both of the remote computing apparatus 140 and the local computing device 160 may include data storage that may allow for access to processing programs or routines and/or one or more other types of data, e.g., for analyzing a plurality of electrical signals captured by the electrode apparatus 110, for determining a patient's cardiac condition, for determining whether a patient would qualify as a candidate for cardiac therapy, for determining EHI, for determining QRS onsets, QRS offsets, medians, modes, averages, peaks or maximum values, valleys or minimum values, for determining electrical activation times, for driving a graphical user interface configured to noninvasively assist a user in determining a patient's cardiac condition and whether the patient may benefit from cardiac therapy, etc.

The remote computing apparatus 140 may be operatively coupled to the input apparatus 142 and the display apparatus 130 to, e.g., transmit data to and from each of the input apparatus 142 and the display apparatus 130, and the local computing device 160 may be operatively coupled to the input apparatus 162 and the display apparatus 170 to, e.g., transmit data to and from each of the input apparatus 162 and the display apparatus 170. For example, the remote computing apparatus 140 and the local computing device 160 may be electrically coupled to the input apparatus 142, 162 and the display apparatus 130, 170 using, e.g., analog electrical connections, digital electrical connections, wireless connections, bus-based connections, network-based connections, internet-based connections, etc. As described further herein, a user may provide input to the input apparatus 142, 162 to view and/or select one or more pieces of configuration information related to the cardiac therapy delivered by cardiac therapy apparatus such as, e.g., an implantable medical device.

Each of the remote computing apparatus 140 and local computing device may include a communication interface. The communication interface of the remote computing apparatus 140 may be referred to as the remote communication interface, and the communication interface of the local computing device 160 may be referred to as the local communication interface. The communication interfaces of the remote computing apparatus 140 and the local computing device 160 may be used to communicate with other devices and apparatus such as the electrode apparatus 110 and each other. In one embodiment, the communication interfaces may include a transceiver and antenna for wirelessly communicating with an external device using radio frequency (RF) communication or other communication protocols. Further, the communication interfaces may be configured to be unidirectional or bi-directional.

Although as depicted the input apparatus 142 is a keyboard and the input apparatus 162 is a touchscreen, it is to be understood that the input apparatus 142, 162 may include any apparatus capable of providing input to the remote computing apparatus 140 and the computing device 160 to perform the functionality, methods, and/or logic described herein. For example, the input apparatus 142, 162 may include a keyboard, a mouse, a trackball, a touchscreen (e.g., capacitive touchscreen, a resistive touchscreen, a multi-touch touchscreen, etc.), etc. Likewise, the display apparatus 130, 170 may include any apparatus capable of displaying information to a user, such as a graphical user interface 132, 172 including electrode status information, cardiac condition information, cardiac therapy benefit information, graphical maps of electrical activation, a plurality of signals for the external electrodes over one or more heartbeats, QRS complexes, various cardiac therapy scenario selection regions, various rankings of cardiac therapy scenarios, various pacing parameters, electrical heterogeneity information (EHI), textual instructions, graphical depictions of anatomy of a human heart, images or graphical depictions of the patient's heart, graphical depictions of locations of one or more electrodes, graphical depictions of a human torso, images or graphical depictions of the patient's torso, graphical depictions or actual images of implanted electrodes and/or leads, etc. Further, the display apparatus 130, 170 may include a liquid crystal display, an organic light-emitting diode screen, a touchscreen, a cathode ray tube display, etc.

The processing programs or routines stored and/or executed by the remote computing apparatus 140 and the local computing device 160 may include programs or routines for computational mathematics, matrix mathematics, decomposition algorithms, compression algorithms (e.g., data compression algorithms), calibration algorithms, image construction algorithms, signal processing algorithms (e.g., various filtering algorithms, Fourier transforms, fast Fourier transforms, etc.), standardization algorithms, comparison algorithms, vector mathematics, or any other processing used to implement one or more illustrative methods and/or processes described herein. Data stored and/or used by the remote computing apparatus 140 and the local computing device 160 may include, for example, electrical signal/waveform data from the electrode apparatus 110 (e.g., a plurality of QRS complexes), electrical activation times from the electrode apparatus 110, EHI, cardiac sound/signal/waveform data from acoustic sensors, graphics (e.g., graphical elements, icons, buttons, windows, dialogs, pull-down menus, graphic areas, graphic regions, 3D graphics, etc.), graphical user interfaces, results from one or more processing programs or routines employed according to the disclosure herein (e.g., electrical signals, electrical heterogeneity information, etc.), or any other data that may be used for carrying out the one and/or more processes or methods described herein.

In one or more embodiments, the illustrative systems, methods, devices, and interfaces may be implemented using one or more computer programs executed on programmable computers, such as computers that include, for example, processing capabilities, data storage (e.g., volatile or non-volatile memory and/or storage elements), input devices, and output devices. Program code and/or logic described herein may be applied to input data to perform functionality described herein and generate desired output information. The output information may be applied as input to one or more other devices and/or methods as described herein or as would be applied in a known fashion.

The one or more programs used to implement the systems, methods, devices, and/or interfaces described herein may be provided using any programmable language, e.g., a high-level procedural and/or object orientated programming language that is suitable for communicating with a computer system. Any such programs may, for example, be stored on any suitable device, e.g., a storage media, that is readable by a general or special purpose program running on a computer system (e.g., including processing apparatus) for configuring and operating the computer system when the suitable device is read for performing the procedures described herein. In other words, at least in one embodiment, the illustrative systems, methods, devices, and interfaces may be implemented using a computer readable storage medium, configured with a computer program, where the storage medium so configured causes the computer to operate in a specific and predefined manner to perform functions described herein. Further, in at least one embodiment, the illustrative systems, methods, devices, and interfaces may be described as being implemented by logic (e.g., object code) encoded in one or more nontransitory media that includes code for execution and, when executed by a processor or processing circuitry, is operable to perform operations such as the methods, processes, and/or functionality described herein.

The remote computing apparatus 140 and the local computing device 160 may be, for example, any fixed or mobile computer system (e.g., a controller, a microcontroller, a personal computer, minicomputer, tablet computer, smartphone, etc.). The exact configurations of the remote computing apparatus 140 and the local computing device 160 are not limiting, and essentially any device including processing circuitry capable of providing suitable computing capabilities and control capabilities (e.g., signal analysis, mathematical functions such as medians, modes, averages, maximum value determination, minimum value determination, slope determination, minimum slope determination, maximum slope determination, graphics processing, etc.) may be used. As described herein, a digital file may be any medium (e.g., volatile or non-volatile memory, a CD-ROM, a punch card, magnetic recordable tape, etc.) containing digital bits (e.g., encoded in binary, trinary, etc.) that may be readable and/or writeable by the remote computing apparatus 140 and the local computing device 160 described herein. Also, as described herein, a file in user-readable format may be any representation of data (e.g., ASCII text, binary numbers, hexadecimal numbers, decimal numbers, graphically, etc.) presentable on any medium (e.g., paper, a display, etc.) readable and/or understandable by a user. The processing circuitry of each of remote computing apparatus 140 and the local computing device 160 may be operably coupled to the communication interface such that the processing circuitry can communication the electrode apparatus 110, each other, and other devices/apparatus.

In view of the above, it will be readily apparent that the functionality as described in one or more embodiments according to the present disclosure may be implemented in any manner as would be known to one skilled in the art. As such, the computer language, the computer system, or any other software/hardware which is to be used to implement the processes described herein shall not be limiting on the scope of the systems, processes, or programs (e.g., the functionality provided by such systems, processes, or programs) described herein.

The illustrative electrode apparatus 110 may be configured to measure body-surface potentials of a patient 14 and, more particularly, torso-surface potentials of a patient 14. As shown in FIG. 2, the illustrative electrode apparatus 110 may include a set, or array, of external electrodes 112, a strap 113, and interface/amplifier circuitry 116. The electrodes 112 may be attached, or coupled, to the strap 113 and the strap 113 may be configured to be wrapped around the torso of a patient 14 such that the electrodes 112 surround the patient's heart. As further illustrated, the electrodes 112 may be positioned around the circumference of a patient 14, including the posterior, lateral, posterolateral, anterolateral, and anterior locations of the torso of a patient 14.

The illustrative electrode apparatus 110 may be further configured to measure, or monitor, sounds from at least one or both the patient 14. As shown in FIG. 2, the illustrative electrode apparatus 110 may include a set, or array, of acoustic sensors 120 attached, or coupled, to the strap 113. The strap 113 may be configured to be wrapped around the torso of a patient 14 such that the acoustic sensors 120 surround the patient's heart. As further illustrated, the acoustic sensors 120 may be positioned around the circumference of a patient 14, including the posterior, lateral, posterolateral, anterolateral, and anterior locations of the torso of a patient 14.

Further, the electrodes 112 and the acoustic sensors 120 may be electrically connected to interface/amplifier circuitry 116 via wired connection 118. The interface/amplifier circuitry 116 may be configured to amplify the signals from the electrodes 112 and the acoustic sensors 120 and provide the signals to one or both of the remote computing apparatus 140 and the local computing device 160. Other illustrative systems may use a wireless connection to transmit the signals sensed by electrodes 112 and the acoustic sensors 120 to the interface/amplifier circuitry 116 and, in turn, to one or both of the remote computing apparatus 140 and the local computing device 160, e.g., as channels of data. In one or more embodiments, the interface/amplifier circuitry 116 may be electrically coupled to the remote computing apparatus 140 using, e.g., analog electrical connections, digital electrical connections, wireless connections, bus-based connections, network-based connections, internet-based connections, etc.

Although in the example of FIG. 2 the electrode apparatus 110 includes a strap 113, in other examples any of a variety of mechanisms, e.g., tape or adhesives, may be employed to aid in the spacing and placement of electrodes 112 and the acoustic sensors 120. In some examples, the strap 113 may include an elastic band, strip of tape, or cloth. Further, in some examples, the strap 113 may be part of, or integrated with, a piece of clothing such as, e.g., a t-shirt. In other examples, the electrodes 112 and the acoustic sensors 120 may be placed individually on the torso of a patient 14. Further, in other examples, one or both of the electrodes 112 (e.g., arranged in an array) and the acoustic sensors 120 (e.g., also arranged in an array) may be part of, or located within, patches, vests, and/or other manners of securing the electrodes 112 and the acoustic sensors 120 to the torso of the patient 14. Still further, in other examples, one or both of the electrodes 112 and the acoustic sensors 120 may be part of, or located within, two sections of material or two patches. One of the two patches may be located on the anterior side of the torso of the patient 14 (to, e.g., monitor electrical signals representative of the anterior side of the patient's heart, measure surrogate cardiac electrical activation times representative of the anterior side of the patient's heart, monitor or measure sounds of the anterior side of the patient, etc.) and the other patch may be located on the posterior side of the torso of the patient 14 (to, e.g., monitor electrical signals representative of the posterior side of the patient's heart, measure surrogate cardiac electrical activation times representative of the posterior side of the patient's heart, monitor or measure sounds of the posterior side of the patient, etc.). And still further, in other examples, one or both of the electrodes 112 and the acoustic sensors 120 may be arranged in a top row and bottom row that extend from the anterior side of the patient 14 across the left side of the patient 14 to the posterior side of the patient 14.

Yet still further, in other examples, one or both of the electrodes 112 and the acoustic sensors 120 may be arranged in a curve around the armpit area and may have an electrode/sensor-density that less dense on the right thorax that the other remaining areas. For example, the strap 113 can be optimized to form a C-shape for covering areas of torso on the left anterior and left posterior aspects to gather information on left ventricular activation. Further, for example, the strap 113 can be optimized to form a C-shape for application to the right side of the torso to gather information on right ventricular activation. Also, the strap 110 may have a C-shaped design with clearly delineated anatomic markers (e.g., mid-sternal line, left anterior axillary line, posterior vertebral line, etc.) to aid in placing it on the torso.

Additionally, the electrode apparatus 110 can be a reusable belt that may be used outside of a medical clinic in follow-up setting or at patient's home. Thus, the electrode apparatus 110 may be used with a patient's smartphone as the local computing device 160 to measure cardiac electrical activity about the patient's torso to be used to, e.g., determine the patient's cardiac condition, determine whether the patient is a candidate for cardiac therapy, etc.

The electrodes 112 may be configured to surround the heart of the patient 14 and record, or monitor, the electrical signals associated with the depolarization and repolarization of the heart after the signals have propagated through the torso of a patient 14. Each of the electrodes 112 may be used in a unipolar configuration to sense the torso-surface potentials that reflect the cardiac signals. The interface/amplifier circuitry 116 may also be coupled to a return or indifferent electrode (not shown) that may be used in combination with each electrode 112 for unipolar sensing.

In some examples, there may be about 12 to about 50 electrodes 112 and about 12 to about 50 acoustic sensors 120 spatially distributed around the torso of a patient. Other configurations may have more or fewer electrodes 112 and more or fewer acoustic sensors 120. It is to be understood that the electrodes 112 and acoustic sensors 120 may not be arranged or distributed in an array extending all the way around or completely around the patient 14. Instead, the electrodes 112 and acoustic sensors 120 may be arranged in an array that extends only part of the way or partially around the patient 14. For example, the electrodes 112 and acoustic sensors 120 may be distributed on the anterior, posterior, and left sides of the patient with less or no electrodes and acoustic sensors proximate the right side (including posterior and anterior regions of the right side of the patient).

One or both of the local computing device 160 and the remote computing apparatus 140 may record and analyze the torso-surface potential signals sensed by electrodes 112 and the sound signals sensed by the acoustic sensors 120, which are amplified/conditioned by the interface/amplifier circuitry 116. One or both of the local computing device 160 and the remote computing apparatus 140 may be configured to analyze the electrical signals from the electrodes 112 to provide electrocardiogram (ECG) signals, information, or data from the patient's heart as will be further described herein. One or both of the local computing device 160 and the remote computing apparatus 140 may be configured to analyze the electrical signals from the acoustic sensors 120 to provide sound signals, information, or data from the patient's body.

Additionally, the remote computing apparatus 140 and the local computing device 160 may be configured to provide graphical user interfaces 132, 172 depicting various information related to the electrode apparatus 110 and the data gathered, or sensed, using the electrode apparatus 110. For example, the graphical user interfaces 132, 172 may depict ECGs including QRS complexes obtained using the electrode apparatus 110 and sound data including sound waves obtained using the acoustic sensors 120 as well as other information related thereto. Illustrative systems, devices, and methods may noninvasively use the electrical information collected using the electrode apparatus 110 and the sound information collected using the acoustic sensors 120 to evaluate a patient's cardiac health and to determine whether cardiac therapy may be beneficial for the patient.

Further, the electrode apparatus 110 may further include reference electrodes and/or drive electrodes to be, e.g. positioned about the lower torso of the patient 14, that may be further used by the system 100. For example, the electrode apparatus 110 may include three reference electrodes, and the signals from the three reference electrodes may be combined to provide a reference signal. Further, the electrode apparatus 110 may use of three caudal reference electrodes (e.g., instead of standard references used in a Wilson Central Terminal) to get a "true" unipolar signal with less noise from averaging three caudally located reference signals.

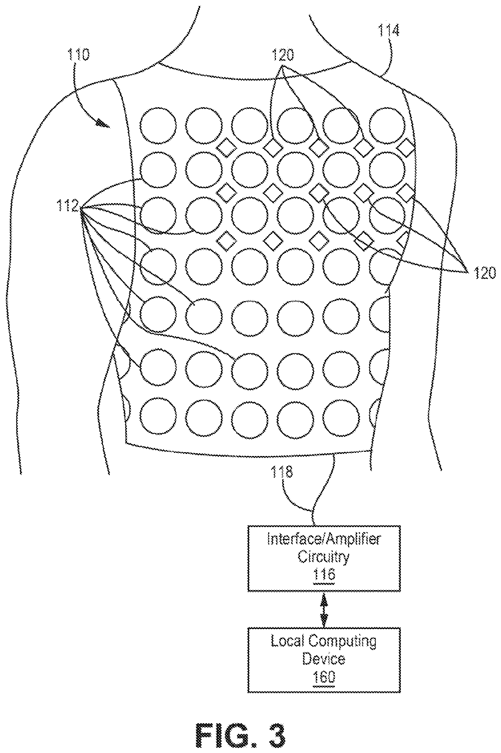

FIG. 3 illustrates another illustrative electrode apparatus 110 that includes a plurality of electrodes 112 configured to surround the heart of the patient 14 and record, or monitor, the electrical signals associated with the depolarization and repolarization of the heart after the signals have propagated through the torso of the patient 14 and a plurality of acoustic sensors 120 configured to surround the heart of the patient 14 and record, or monitor, the sound signals associated with the heart after the signals have propagated through the torso of the patient 14. The electrode apparatus 110 may include a vest 114 upon which the plurality of electrodes 112 and the plurality of acoustic sensors 120 may be attached, or to which the electrodes 112 and the acoustic sensors 120 may be coupled. In at least one embodiment, the plurality, or array, of electrodes 112 may be used to collect electrical information such as, e.g., surrogate electrical activation times. Similar to the electrode apparatus 110 of FIG. 2, the electrode apparatus 110 of FIG. 3 may include interface/amplifier circuitry 116 electrically coupled to each of the electrodes 112 and the acoustic sensors 120 through a wired connection 118 and be configured to transmit signals from the electrodes 112 and the acoustic sensors 120 to remote computing apparatus 140. As illustrated, the electrodes 112 and the acoustic sensors 120 may be distributed over the torso of a patient 14, including, for example, the posterior, lateral, posterolateral, anterolateral, and anterior locations of the torso of a patient 14.

The vest 114 may be formed of fabric with the electrodes 112 and the acoustic sensors 120 attached to the fabric. The vest 114 may be configured to maintain the position and spacing of electrodes 112 and the acoustic sensors 120 on the torso of the patient 14. Further, the vest 114 may be marked to assist in determining the location of the electrodes 112 and the acoustic sensors 120 on the surface of the torso of the patient 14. In some examples, there may be about 25 to about 256 electrodes 112 and about 25 to about 256 acoustic sensors 120 distributed around the torso of the patient 14, though other configurations may have more or fewer electrodes 112 and more or fewer acoustic sensors 120.

The illustrative systems, methods, and devices may be used to provide noninvasive assistance to a user in the evaluation of a patient's cardiac health and/or evaluation whether the patient may benefit from cardiac therapy. For example, the illustrative systems, methods, and devices may be used to assist a user acquiring cardiac electrical activity in-home and analyzing the acquired cardiac electrical activity to determine the patient's cardiac condition and/or determine whether the patient may benefit from cardiac therapy.

Further, it is to be understood that the remote computing apparatus 140 and the local computing device 160 may be operatively coupled to each other in a plurality of different ways using their respective communication interfaces so as to perform, or execute, the functionality described herein. For example, in the embodiment depicted, the computing device 140 may be wireless operably coupled to the local computing device 160 as depicted by the wireless signal lines emanating therebetween. Additionally, as opposed to wireless connections, one or more of the remote computing apparatus 140 and the remoting computing device 160 may be operably coupled through one or wired electrical connections.

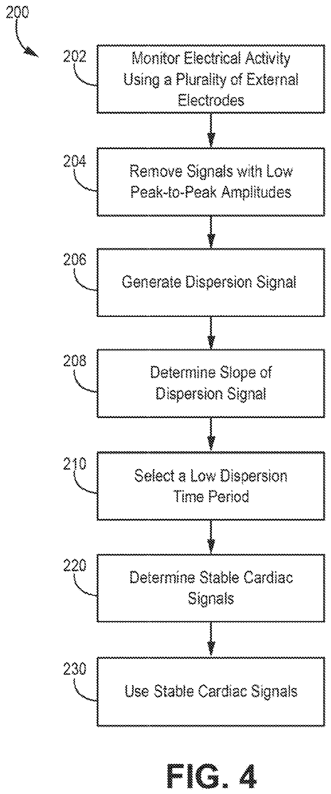

An illustrative method 200 of identifying stable cardiac signals is depicted in FIG. 4. The illustrative method 200 may be generally described to be used in the evaluation of a plurality of cardiac signals (ECG signals) monitored using a plurality of external electrodes such as those described herein with respect to FIGS. 1-3. Once the cardiac signals are evaluated to determine which are stable or non-stable, the stable cardiac signals may be used for other purposes such as detecting QRS complexes, identifying QRS onsets, identifying QRS offsets, noninvasively evaluating a patient's natural, intrinsic cardiac condition, etc.

The method 200 may include monitoring, or measuring, electrical activity using a plurality of external electrodes 202. The plurality of external electrodes may be similar to the external electrodes provided by the electrode apparatus 110 as described herein with respect to FIGS. 1-3. For example, the plurality of external electrodes may be part, or incorporated into, a vest or band that is located about a patient's torso. More specifically, the plurality of electrodes may be described as being surface electrodes positioned in an array configured to be located proximate the skin of the torso of a patient.

Monitoring, or measuring, electrical activity using a plurality of external electrodes 202 may result in, or provide, a plurality of cardiac signals (ECG signals), each cardiac signal corresponding to one of the external electrodes spatially distributed about the patient. The cardiac signals, or electrical activity, may be monitored for a period of time. The period of time, which the cardiac signals may be monitored, may be referred to as an analysis time period. In at least one embodiment, the length of the analysis time period may be selected to ensure capture of a cardiac cycle. In at least one embodiment, the length of the analysis time period may be selected to ensure capture of a portion of a cardiac cycle such as a QRS complex. In at least one embodiment, the analysis time period may be about 1 second. Further, the analysis time period may be between about 0.75 seconds to about 2.0 seconds. For example, the analysis time period may be greater than or equal to 0.75 seconds, greater than or equal to 0.85 seconds, greater than or equal to 0.95 seconds, etc. and/or less than or equal to about 2.0 seconds, less than or equal to about 1.5 seconds, less than or equal to about 1.25 seconds, etc. In at least one embodiment, it may be described that multiple simultaneous body surface electrode signals may be collected for 1 second.

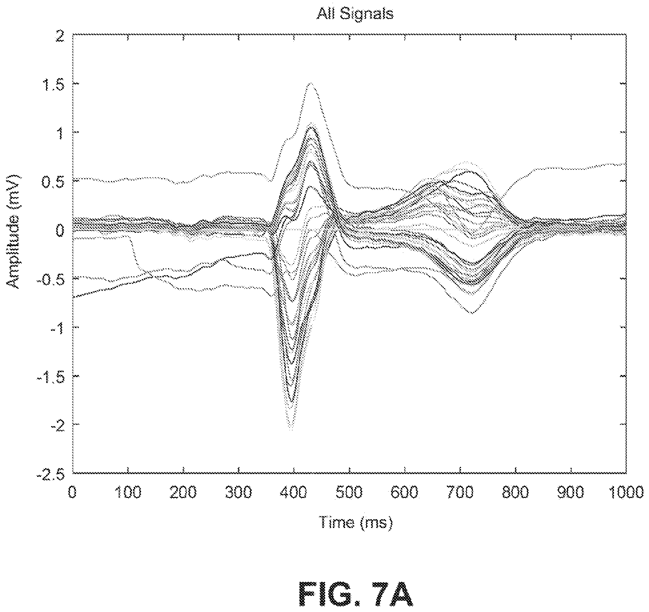

Thus, the monitoring process 202 may result in a plurality of cardiac signals over an analysis time period. A graph of a plurality of illustrative monitored cardiac signals (e.g., 37 signals) over a 1-second analysis time period is depicted in FIG. 7A. The plurality of cardiac signals may include stable signals and unstable signals. To identify, and subsequently, remove the unstable cardiac signals, the method 200 may first remove signals with low peak-to-peak amplitudes 204. The signals with low peak-to-peak amplitudes may be of non-substantial value when creating a dispersion signal, and such signals with low peak-to-peak amplitudes may be described as dampening the impact of the signals with meaningful morphology. Additionally, the signals with low peak-to-peak amplitudes may be measured using electrodes with poor or no contact. Thus, the low peak-to-peak amplitudes are removed.

In at least one embodiment, removing signals with low peak-to-peak amplitudes 204 may include generating a peak-to-peak amplitude for each of the plurality of cardiac signals within the analysis time period. The peak-to-peak amplitude may be described as the amplitude or vertical distance between the highest, or maximum, voltage and the lowest, or minimum, voltage of the cardiac signal over the analysis time period. In other words, a difference may be calculated between the highest voltage and the lowest voltage of the cardiac signal over the analysis time period resulting in a peak-to-peak amplitude value.

The peak-to-peak amplitude of each cardiac signal may be analyzed to determine if the cardiac signal is classified as being low amplitude, and if the cardiac signal is classified as low amplitude, the low amplitude cardiac signal will be removed from the plurality of cardiac signals used for further analysis. In at least one embodiment, a low amplitude threshold may be utilized. For example, if the peak-to-peak amplitude of the cardiac signal within the analysis time period is less than or equal to a low amplitude threshold, then it may be determined that the cardiac signal is low amplitude. Conversely, for example, if the peak-to-peak amplitude of the cardiac signal within the analysis time period is greater than the low amplitude threshold, then it may be determined that the cardiac signal is not low amplitude. The low amplitude threshold may be between about 0.05 millivolts (mV) and about 1.0 mV. In at least one embodiment, the low amplitude threshold is 0.12 mV. In other words, cardiac signals with small amplitude across the 1-second time frame may be identified, and subsequently, removed.

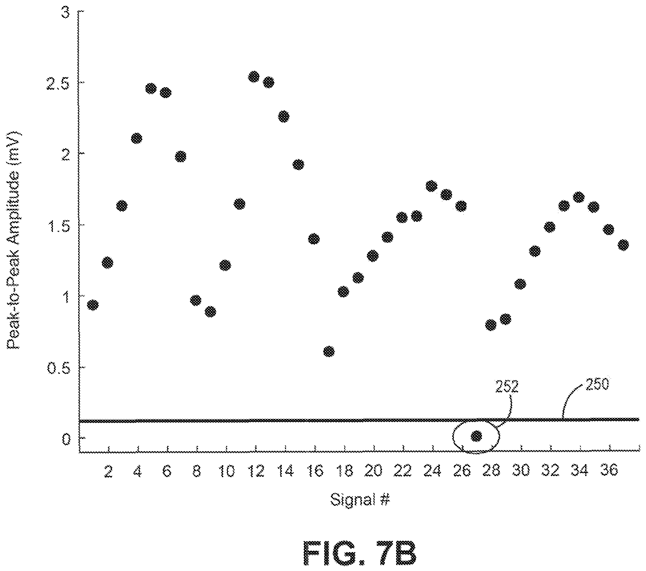

A plurality of peak-to-peak amplitudes corresponding to the plurality of cardiac signals of FIG. 7A are plotted in the graph depicted in FIG. 7B. In this example, the low amplitude threshold is 0.12 millivolts, which is represented by horizontal line 250. As shown, a single peak-to-peak amplitude is less than or equal to the is 0.12 millivolt threshold as indicated by circle 252. Thus, the low amplitude cardiac signal corresponding to the peak-to-peak amplitude circled and below the low amplitude threshold may be removed from the plurality of cardiac signals further used by the illustrative methods and processes described herein.

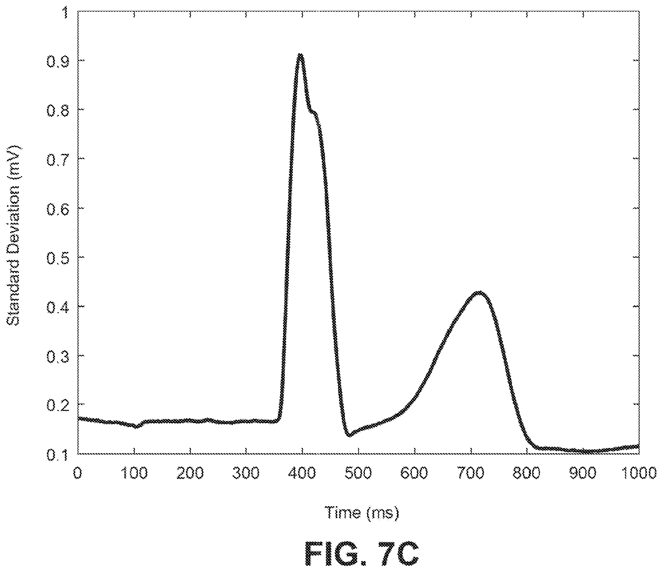

A dispersion signal may then be generated based on the plurality of cardiac signals 206 (e.g., the remaining plurality of signals after the low amplitude signals have been removed in 204). The dispersion signal is representative of the dispersion of the plurality of cardiac signals over the analysis time period. In other words, the dispersion signal may be calculated, or determined, across all signals at each time point over the analysis time period. In at least one embodiment, the dispersion signal is a standard deviation. In other words, generating a dispersion signal from the plurality of cardiac signals may include, or be, determining a standard deviation of the plurality of cardiac signals over the analysis time period. Further, it may be described that the standard deviation across all signals may be calculated at each sampled time point. An illustrative dispersion signal generated from the plurality of cardiac signals of FIG. 7A after the single low amplitude cardiac signal was removed as described with respect to FIG. 7B is plotted on the graph depicted in FIG. 7C.

The rate of change, or slope, of the dispersion signal may be utilized in the illustrative method 200 to identify a smaller period of time within the analysis time period to analyze each of the cardiac signals to determine whether or not such cardiac signals are stable. Generally, it may be described that the smaller period of time within the analysis time period may be identified so as to find the "true" baseline of the signals (e.g., to exclude various morphological features corresponding to cardiac events with the cardiac cycle such as the P-wave, QRS complex, T-wave, etc.). Thus, the method 200 may generate, or calculate, a rate of change, or slope, of the dispersion signal 208. In particular, it may be described that a first derivative of the dispersion signal may be determined. Further, it may be described that the slope of the standard deviation signal may be calculated across each sampled time point.

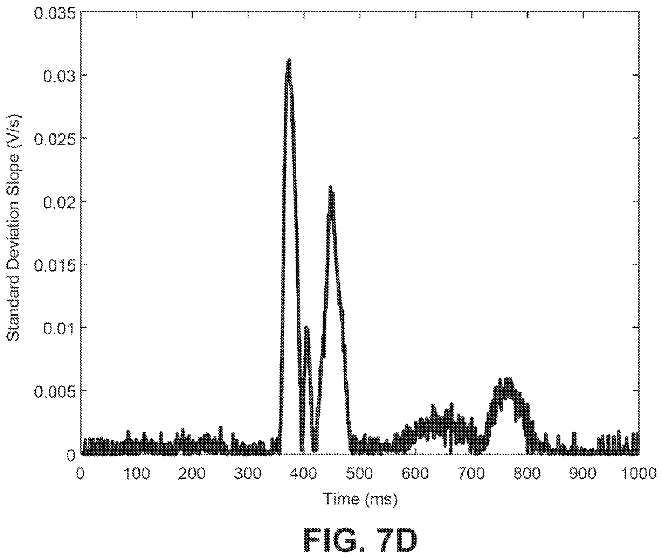

An illustrative slope generated from the dispersion signal of FIG. 7C is plotted on the graph depicted in FIG. 7D. Although the illustrative method 200 describes generating a slope, it is to be understood that illustrative methods and processes may compute the slope, or rate of change, of the dispersion signal "on-the-fly" while performing subsequent processes or may integrate computation of the slope, or rate of change, of the dispersion within further processes.

Using the rate of change, or slope, of the dispersion signal, the method 200 may next select, or identify, a low dispersion time period within the analysis time period 210. For example, a minimum, or lowest, rate of change of the dispersion signal over a sliding window within the analysis time period may be determined, and then the low dispersion time period may be determined thereon. The sliding window may be between about 50 milliseconds (ms) and about 350 ms. In at least one embodiment, the sliding window is 200 ms. In other words, the 200 ms in which the standard deviation slope changes the least may be identified. More specifically, a rolling summation of the rate of change, or slope, of the dispersion over the sliding window may be determined or generated, and then, the minimum rolling summation within the analysis time period may be identified. In other words, a sliding window may be "slid" over the slope of the dispersion signal across the analysis time period, and the summation value within the sliding window may be computed.

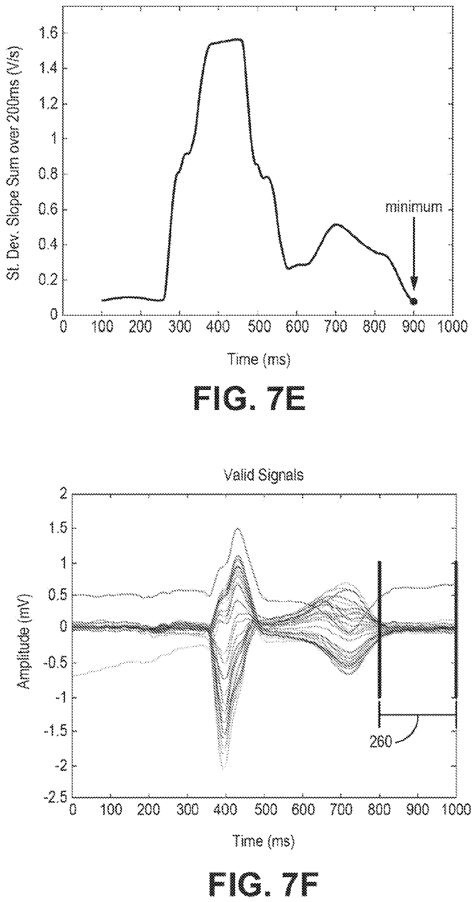

A summation over a 200 ms sliding window of the slope of the dispersion signal of FIG. 7D is plotted and the minimum rolling summation is identified in the graph depicted in FIG 7E. The minimum rolling summation may be used to define the center, or midpoint, of the low dispersion time period. As shown in FIG. 7E, a minimum, or lowest, rolling summation is identified at 900 ms. Thus, an illustrative low dispersion time period may be selected, or identified, based on the 900 ms minimum, or lowest, rolling summation. For example, a low dispersion time period 260 is identified on the plurality of cardiac signals plotted on the graph depicted in FIG 7F using the 900 ms minimum rolling summation. In particular, a 200 ms low dispersion time period is identified using the 900 ms minimum rolling summation as a midpoint.

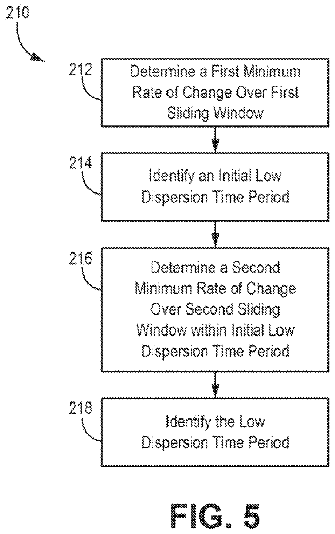

Selection, or identification, of the low dispersion time period within the analysis time period 210, however, may be a two-step process, where a first, initial low dispersion time period is selected, and then a second, final low dispersion time period is selected within the first, initial low dispersion time period. Another illustrative method of selecting a low dispersion time period 210 is depicted in FIG. 5, which utilizes a two-step process to select the low dispersion time period.

First, a first minimum rate of change, or slope, of a first sliding window may be determined 212, and an initial low dispersion time period may be identified 214. Such processes 212, 214 may be substantially the same as already described herein and with reference to FIGS. 7E-7F, and as such, will not be described further herein. Next, however, a second minimum rate of change, or slope, of second sliding window within the already-identified, initial low dispersion time period may be determined 216 (similar to as described herein with respect to process 212 but now only within the initial low dispersion time period), and then the final, or ultimate, low dispersion time period may be selected based thereon 218. Again, such processes 216, 218 may be substantially the same as already described herein and with reference to FIGS. 7E-7F, and as such, will not be described further herein.

The second sliding window is less than the first sliding window. In other words, the second sliding window is shorter period of time than the first sliding window (e.g., so as to be able to analyze or evaluate small chunks of time within the initial low dispersions time period). Thus, the second sliding window, and process associated therewith, may more closely analyze the data within the initial low dispersion time period to determine the best time period therein for further analysis of signal stability. The second sliding window may be between about 25 milliseconds (ms) and about 150 ms. In at least one embodiment, the second sliding window is 100 ms.

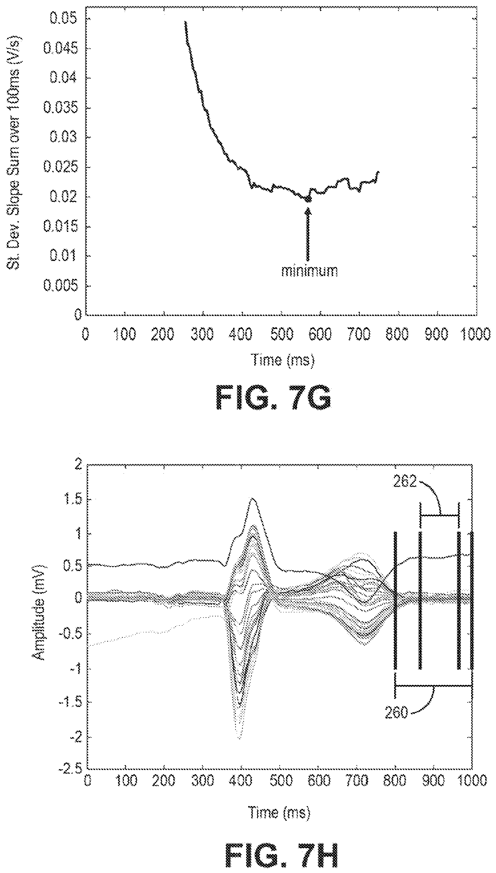

These second sliding window processes are shown in FIGS. 7G-7H. A summation over a second sliding window of the slope of the dispersion signal within the low dispersion time period of FIG. 7F is plotted in the graph of FIG. 7G. The minimum rolling summation may define the center, or midpoint, of the low dispersion time period. As shown in FIG. 7G, a minimum, or lowest, rolling summation is identified at 916 ms. Thus, the final, low dispersion time period may be identified based on the 916 ms minimum, or lowest, rolling summation. For example, the low dispersion time period 262 is identified using the summation over the second sliding window of the slope of the dispersion signal within the initial, low dispersion time period 260 of FIGS. 7F-G on the plurality of cardiac signals plotted on the graph depicted in FIG 7H. In this example, the initial low dispersion time period is 200 ms and the low dispersion time period is 100 ms, and thus, it may be described that the 100 ms within the 200ms window in which the standard deviation slope changes the least may be identified.

The illustrative method 200 further includes determining whether each of the plurality of cardiac signals is stable 220. The determination of whether each cardiac signal is stable may utilize the portion, or time period, of the cardiac signal within the selected low dispersion time period 210. In other words, only the portion or segment of each cardiac signal monitored, or recorded, over the low dispersion time period may be utilized to determine whether or not the cardiac signal is stable.

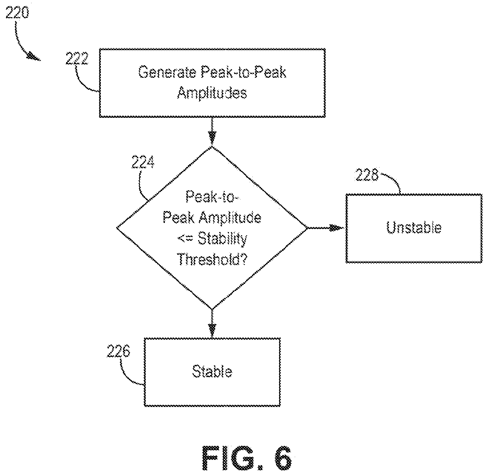

Each cardiac signal may be analyzed for stability over the low dispersion time period using various processes. One illustrative example that is depicted in FIG. 6 may analysis the peak-to-peak amplitudes of each of the cardiac signals over the low dispersion time period to determine whether the cardia signal is stable or unstable. As shown, the illustrative process 220 may include generating a peak-to-peak amplitude for each of the plurality of cardiac signals within the analysis time period 222. The peak-to-peak amplitude may be described as the amplitude or vertical distance between the highest, or maximum, voltage and the lowest, or minimum, voltage of the cardiac signal over the low dispersion time period. In other words, a difference may be calculated between the highest voltage and the lowest voltage of the cardiac signal over the low dispersion time period resulting in a peak-to-peak amplitude value.

The peak-to-peak amplitude of each cardiac signal may then be analyzed to determine if the cardiac signal is stable or unstable. As described further herein, if the cardiac signal is unstable, it will be removed from the plurality of cardiac signals used for further analysis (e.g., to detect QRS complexes, to determine QRS onsets and offsets, etc.). As shown, a stability threshold may be utilized. If the peak-to-peak amplitude of the cardiac signal within the low dispersion time period is less than or equal to the stability amplitude threshold 224, then it may be determined that the cardiac signal is stable 226. Conversely, if the peak-to-peak amplitude of the cardiac signal within the low dispersion time period is greater than the stability amplitude threshold 224, then it may be determined that the cardiac signal is unstable 228.

The stability threshold may be set, or determined, based on the plurality of generated peak-to-peak amplitudes of the cardiac signals within the low dispersion time period. For instance, one or more composite values may be generated or determined based on the plurality of generated peak-to-peak amplitudes of the cardiac signals within the low dispersion time period, which may be used to determine or identify outliers indicative of unstable signals. In at least one embodiment, a median value may be determined based on the plurality of generated peak-to-peak amplitudes of the cardiac signals within the low dispersion time period. For example, the stability threshold may be 2.5 times the median peak-to-peak amplitude of the plurality of cardiac signals within the low dispersion time period. Further, for example, the stability threshold may be between about 1.5 times to about 4 times the median peak-to-peak amplitude of the plurality of cardiac signals within the low dispersion time period. In other embodiments, an average or another statistical metric may be used to determine the stability threshold based on the plurality of generated peak-to-peak amplitudes of the cardiac signals within the low dispersion time period.

Further, in other embodiments, the stability threshold may be a fixed value (e.g., based on population studies, etc.). For example, the stability threshold may be between about 0.05 mV and about 0.15. mV. In at least one embodiment, the stability threshold is 0.10 mV.

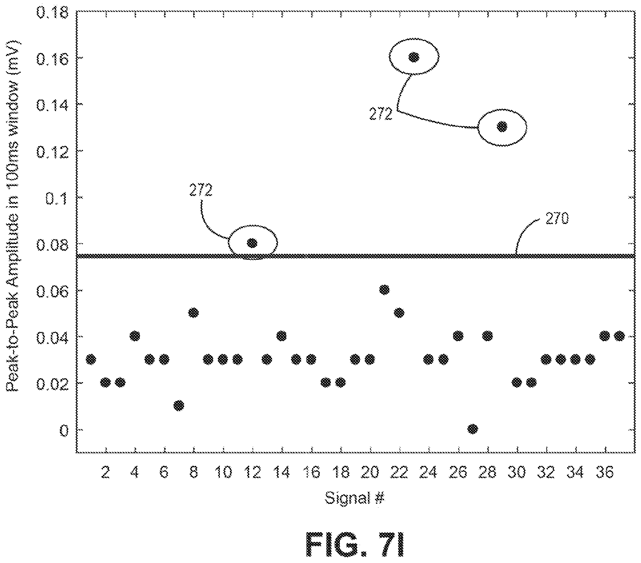

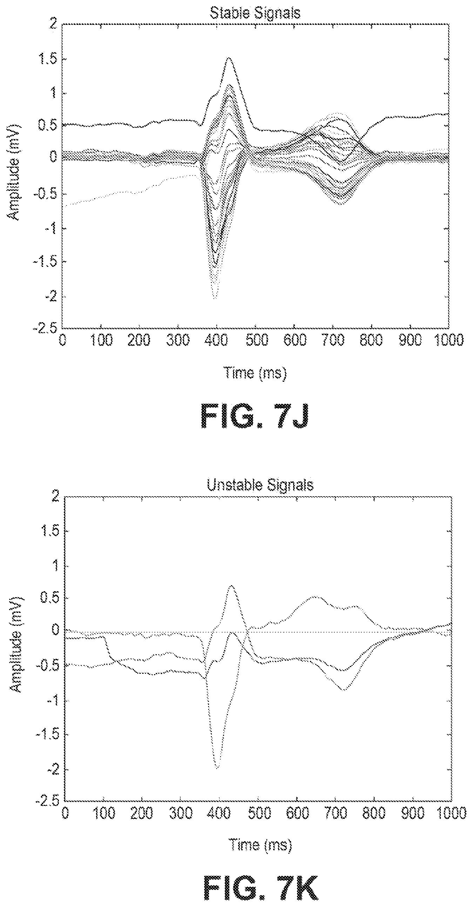

A plurality of peak-to-peak amplitudes corresponding to the plurality of cardiac signals within the low dispersion time period of FIG. 7H are plotted in the graph depicted in FIG. 7I. In this example, the stability threshold is 0.077 millivolts, which is represented by horizontal line 270. As shown, three peak-to-peak amplitudes are greater than the 0.077 millivolt threshold as indicated by circles 272. Thus, the cardiac signals corresponding to the circled peak-to-peak amplitudes and above the stability threshold may be removed from the plurality of cardiac signals further used by the illustrative methods and processes described herein. The result of the processes depicted in FIGS. 7A-7I are shown in FIG. 7J, which depicts a graph of the stable cardiac signals of the plurality of cardiac signals of FIG. 7A, and FIG. 7K, which depicts a graph of the stable cardiac signals of the plurality of cardiac signals of FIG. 7A.

The illustrative method 200 further include utilizing the stable cardiac signals 230. For example, the plurality of cardiac signals plotted on the graph depicted in FIG. 7J may be further used in analysis. In particular, the plurality of stable cardiac signals may be utilized to determine a QRS onset and a QRS offset within the analysis time period. In this way, unstable cardiac signals will not result in errant QRS onset and offset determination. Determination of QRS onsets and QRS offsets may be described in U.S. Pat. App. Pub. No. 2018/0263522 A1 entitled "QRS Offset and Onset Determination" published on Sept. 20, 2018.

After QRS onset and offset is determined or other processes are performed using the stable cardiac signals, further analysis may be performed on the plurality of cardiac signals such as the generation of activation times and electrical heterogeneity information (EHI) or other data based on such activation times. It is to be understood, however, that the unstable cardiac signals may be added back into the data set used to generated activation times and EHI. The EHI may be described as information, or data, representative of at least one of mechanical cardiac functionality and electrical cardiac functionality. The EHI and other cardiac therapy information may be described in U.S. Provisional Patent Application No. 61/834,133 entitled "METRICS OF ELECTRICAL DYSSYNCHRONY AND ELECTRICAL ACTIVATION PATTERNS FROM SURFACE ECG ELECTRODES" and filed on June 12, 2013.

Electrical heterogeneity information (e.g., data) may be defined as information indicative of at least one of mechanical synchrony or dyssynchrony of the heart and/or electrical synchrony or dyssynchrony of the heart. In other words, electrical heterogeneity information may represent a surrogate of actual mechanical and/or electrical functionality of a patient's heart. In at least one embodiment, relative changes in electrical heterogeneity information (e.g., from baseline heterogeneity information to therapy heterogeneity information, from a first set of heterogeneity information to a second set of therapy heterogeneity information, etc.) may be used to determine a surrogate value representative of the changes in hemodynamic response (e.g., acute changes in LV pressure gradients). The left ventricular pressure may be typically monitored invasively with a pressure sensor located in the left ventricular of a patient's heart. As such, the use of electrical heterogeneity information to determine a surrogate value representative of the left ventricular pressure may avoid invasive monitoring using a left ventricular pressure sensor.

In at least one embodiment, the electrical heterogeneity information may include a standard deviation of ventricular activation times measured using some or all of the external electrodes, e.g., of the electrode apparatus 110. Further, local, or regional, electrical heterogeneity information may include standard deviations and/or averages of activation times measured using electrodes located in certain anatomic areas of the torso. For example, external electrodes on the left side of the torso of a patient may be used to compute local, or regional, left electrical heterogeneity information.

The electrical heterogeneity information may be generated using one or more various systems and/or methods. For example, electrical heterogeneity information may be generated using an array, or a plurality, of surface electrodes and/or imaging systems as described in U.S. Pat. App. Pub. No. 2012/0283587 A1 published November 8, 2012 and entitled "ASSESSING INRA-CARDIAC ACTIVATION PATTERNS AND ELECTRICAL DYSSYNCHRONY," U.S. Pat. App. Pub. No. 2012/0284003 A1 published November 8, 2012 and entitled "ASSESSING INTRA-CARDIAC ACTIVATION PATTERNS", and U.S. Pat. No. 8,180,428 B2 issued May 15, 2012 and entitled "METHODS AND SYSTEMS FOR USE IN SELECTING CARDIAC PACING SITES".

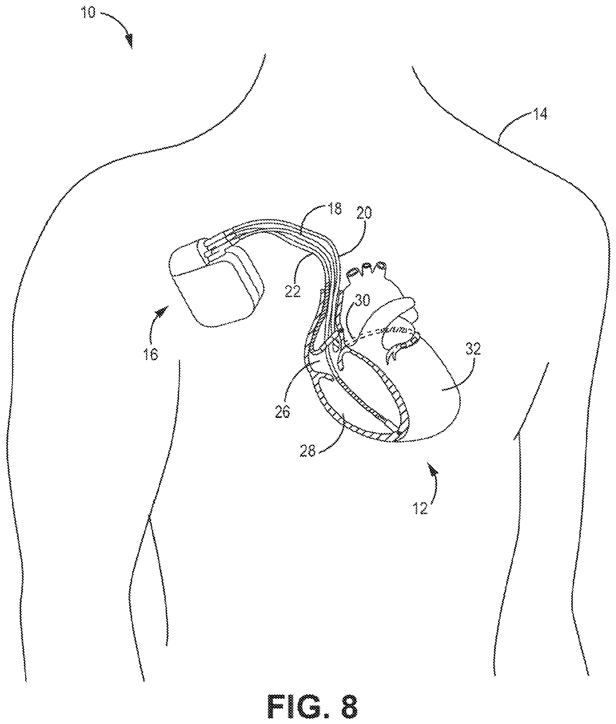

As described herein, the illustrative systems and methods may be used in the processing of data that may ultimately provide an indication of whether a patient may benefit from cardiac therapy or assist in the configuration or tuning of cardiac therapy being delivered to a patient. Such cardiac therapy systems and devices that may be used by patients (after being instructed by the illustrative systems, methods, and devices that they may be candidates for cardiac therapy) are further described herein with reference to FIGS. 8-10.

FIG. 8 is a conceptual diagram illustrating an illustrative therapy system 10 that may be used to deliver pacing therapy to a patient 14. Patient 14 may, but not necessarily, be a human. The therapy system 10 may include an implantable medical device 16 (IMD), which may be coupled to leads 18, 20, 22. The IMD 16 may be, e.g., an implantable pacemaker, cardioverter, and/or defibrillator, that delivers, or provides, electrical signals (e.g., paces, etc.) to and/or senses electrical signals from the heart 12 of the patient 14 via electrodes coupled to one or more of the leads 18, 20, 22.

The leads 18, 20, 22 extend into the heart 12 of the patient 14 to sense electrical activity of the heart 12 and/or to deliver electrical stimulation to the heart 12. In the example shown in FIG. 8, the right ventricular (RV) lead 18 extends through one or more veins (not shown), the superior vena cava (not shown), and the right atrium 26, and into the right ventricle 28. The left ventricular (LV) coronary sinus lead 20 extends through one or more veins, the vena cava, the right atrium 26, and into the coronary sinus 30 to a region adjacent to the free wall of the left ventricle 32 of the heart 12. The right atrial (RA) lead 22 extends through one or more veins and the vena cava, and into the right atrium 26 of the heart 12.

The IMD 16 may sense, among other things, electrical signals attendant to the depolarization and repolarization of the heart 12 via electrodes coupled to at least one of the leads 18, 20, 22. In some examples, the IMD 16 provides pacing therapy (e.g., pacing pulses) to the heart 12 based on the electrical signals sensed within the heart 12. The IMD 16 may be operable to adjust one or more parameters associated with the pacing therapy such as, e.g., A-V delay and other various timings, pulse wide, amplitude, voltage, burst length, etc. Further, the IMD 16 may be operable to use various electrode configurations to deliver pacing therapy, which may be unipolar, bipolar, quadripoloar, or further multipolar. For example, a multipolar lead may include several electrodes that can be used for delivering pacing therapy. Hence, a multipolar lead system may provide, or offer, multiple electrical vectors to pace from. A pacing vector may include at least one cathode, which may be at least one electrode located on at least one lead, and at least one anode, which may be at least one electrode located on at least one lead (e.g., the same lead, or a different lead) and/or on the casing, or can, of the IMD. While improvement in cardiac function as a result of the pacing therapy may primarily depend on the cathode, the electrical parameters like impedance, pacing threshold voltage, current drain, longevity, etc. may be more dependent on the pacing vector, which includes both the cathode and the anode. The IMD 16 may also provide defibrillation therapy and/or cardioversion therapy via electrodes located on at least one of the leads 18, 20, 22. Further, the IMD 16 may detect arrhythmia of the heart 12, such as fibrillation of the ventricles 28, 32, and deliver defibrillation therapy to the heart 12 in the form of electrical pulses. In some examples, IMD 16 may be programmed to deliver a progression of therapies, e.g., pulses with increasing energy levels, until a fibrillation of heart 12 is stopped.

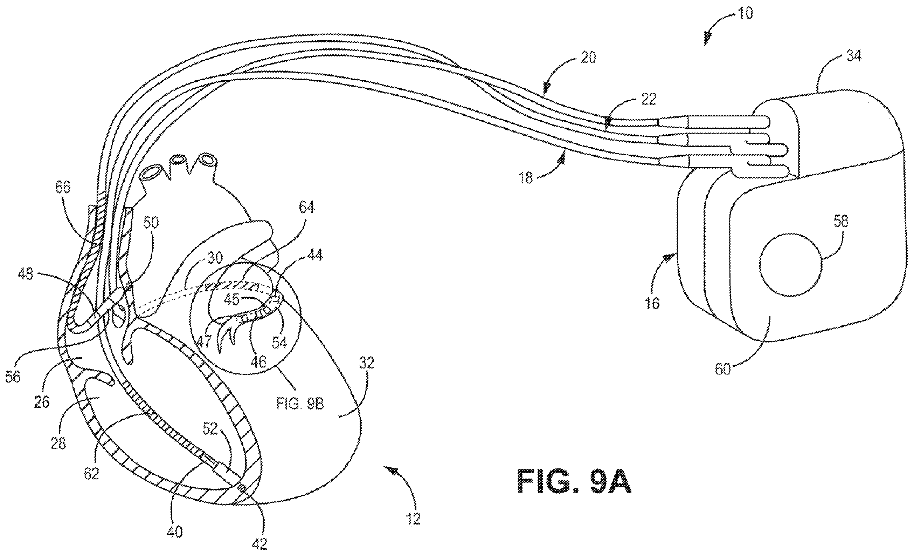

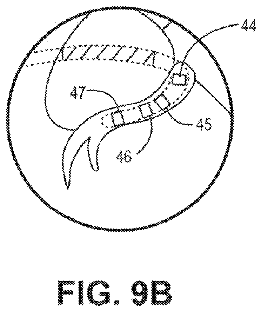

FIGS. 9A-9B are conceptual diagrams illustrating the IMD 16 and the leads 18, 20, 22 of therapy system 10 of FIG. 8 in more detail. The leads 18, 20, 22 may be electrically coupled to a therapy delivery module (e.g., for delivery of pacing therapy), a sensing module (e.g., for sensing one or more signals from one or more electrodes), and/or any other modules of the IMD 16 via a connector block 34. In some examples, the proximal ends of the leads 18, 20, 22 may include electrical contacts that electrically couple to respective electrical contacts within the connector block 34 of the IMD 16. In addition, in some examples, the leads 18, 20, 22 may be mechanically coupled to the connector block 34 with the aid of set screws, connection pins, or another suitable mechanical coupling mechanism.

Each of the leads 18, 20, 22 includes an elongated insulative lead body, which may carry a number of conductors (e.g., concentric coiled conductors, straight conductors, etc.) separated from one another by insulation (e.g., tubular insulative sheaths). In the illustrated example, bipolar electrodes 40, 42 are located proximate to a distal end of the lead 18. In addition, bipolar electrodes 44, 45, 46, 47 are located proximate to a distal end of the lead 20 and bipolar electrodes 48, 50 are located proximate to a distal end of the lead 22.

The electrodes 40, 44, 45, 46, 47, 48 may take the form of ring electrodes, and the electrodes 42, 50 may take the form of extendable helix tip electrodes mounted retractably within the insulative electrode heads 52, 54, 56, respectively. Each of the electrodes 40, 42, 44, 45, 46, 47, 48, 50 may be electrically coupled to a respective one of the conductors (e.g., coiled and/or straight) within the lead body of its associated lead 18, 20, 22, and thereby coupled to a respective one of the electrical contacts on the proximal end of the leads 18, 20, 22.

Additionally, electrodes 44, 45, 46 and 47 may have an electrode surface area of about 5.3 mm² to about 5.8 mm². Electrodes 44, 45, 46, and 47 may also be referred to as LV1, LV2, LV3, and LV4, respectively. The LV electrodes (i.e., left ventricle electrode 1 (LV1) 44, left ventricle electrode 2 (LV2) 45, left ventricle electrode 3 (LV3) 46, and left ventricle 4 (LV4) 47 etc.) on the lead 20 can be spaced apart at variable distances. For example, electrode 44 may be a distance of, e.g., about 21 millimeters (mm), away from electrode 45, electrodes 45 and 46 may be spaced a distance of, e.g. about 1.3 mm to about 1.5 mm, away from each other, and electrodes 46 and 47 may be spaced a distance of, e.g. 20 mm to about 21 mm, away from each other.

The electrodes 40, 42, 44, 45, 46, 47, 48, 50 may further be used to sense electrical signals (e.g., morphological waveforms within electrograms (EGM)) attendant to the depolarization and repolarization of the heart 12. The electrical signals are conducted to the IMD 16 via the respective leads 18, 20, 22. In some examples, the IMD 16 may also deliver pacing pulses via the electrodes 40, 42, 44, 45, 46, 47, 48, 50 to cause depolarization of cardiac tissue of the patient's heart 12. In some examples, as illustrated in FIG. 9A, the IMD 16 includes one or more housing electrodes, such as housing electrode 58, which may be formed integrally with an outer surface of a housing 60 (e.g., hermetically-sealed housing) of the IMD 16 or otherwise coupled to the housing 60. Any of the electrodes 40, 42, 44, 45, 46, 47, 48, 50 may be used for unipolar sensing or pacing in combination with the housing electrode 58. It is generally understood by those skilled in the art that other electrodes can also be selected to define, or be used for, pacing and sensing vectors. Further, any of electrodes 40, 42, 44, 45, 46, 47, 48, 50, 58, when not being used to deliver pacing therapy, may be used to sense electrical activity during pacing therapy.

As described in further detail with reference to FIG. 9A, the housing 60 may enclose a therapy delivery module that may include a stimulation generator for generating cardiac pacing pulses and defibrillation or cardioversion shocks, as well as a sensing module for monitoring the electrical signals of the patient's heart (e.g., the patient's heart rhythm). The leads 18, 20, 22 may also include elongated electrodes 62, 64, 66, respectively, which may take the form of a coil. The IMD 16 may deliver defibrillation shocks to the heart 12 via any combination of the elongated electrodes 62, 64, 66 and the housing electrode 58. The electrodes 58, 62, 64, 66 may also be used to deliver cardioversion pulses to the heart 12. Further, the electrodes 62, 64, 66 may be fabricated from any suitable electrically conductive material, such as, but not limited to, platinum, platinum alloy, and/or other materials known to be usable in implantable defibrillation electrodes. Since electrodes 62, 64, 66 are not generally configured to deliver pacing therapy, any of electrodes 62, 64, 66 may be used to sense electrical activity and may be used in combination with any of electrodes 40, 42, 44, 45, 46, 47, 48, 50, 58. In at least one embodiment, the RV elongated electrode 62 may be used to sense electrical activity of a patient's heart during the delivery of pacing therapy (e.g., in combination with the housing electrode 58, or defibrillation electrode-to-housing electrode vector).

The configuration of the illustrative therapy system 10 illustrated in FIGS. 8-10 is merely one example. In other examples, the therapy system may include epicardial leads and/or patch electrodes instead of or in addition to the transvenous leads 18, 20, 22 illustrated in FIG. 8. Additionally, in other examples, the therapy system 10 may be implanted in/around the cardiac space without transvenous leads (e.g., leadless/wireless pacing systems) or with leads implanted (e.g., implanted transvenously or using approaches) into the left chambers of the heart (in addition to or replacing the transvenous leads placed into the right chambers of the heart as illustrated in FIG. 8). Further, in one or more embodiments, the IMD 16 need not be implanted within the patient 14. For example, the IMD 16 may deliver various cardiac therapies to the heart 12 via percutaneous leads that extend through the skin of the patient 14 to a variety of positions within or outside of the heart 12. In one or more embodiments, the system 10 may utilize wireless pacing (e.g., using energy transmission to the intracardiac pacing component(s) via ultrasound, inductive coupling, RF, etc.) and sensing cardiac activation using electrodes on the can/housing and/or on subcutaneous leads.

In other examples of therapy systems that provide electrical stimulation therapy to the heart 12, such therapy systems may include any suitable number of leads coupled to the IMD 16, and each of the leads may extend to any location within or proximate to the heart 12. For example, other examples of therapy systems may include three transvenous leads located as illustrated in FIGS. 8-10. Still further, other therapy systems may include a single lead that extends from the IMD 16 into the right atrium 26 or the right ventricle 28, or two leads that extend into a respective one of the right atrium 26 and the right ventricle 28.

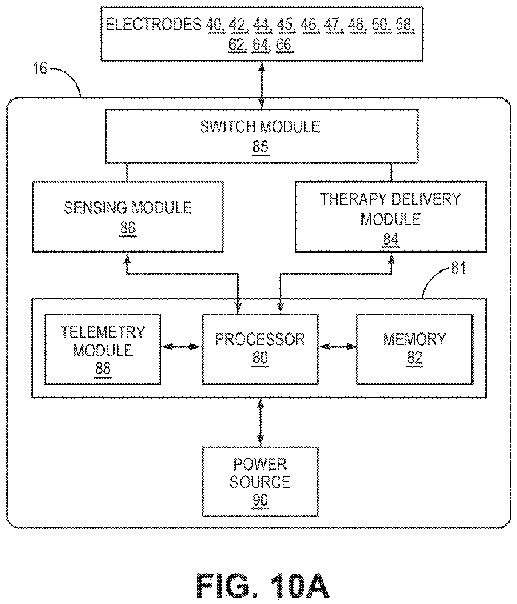

FIG. 10A is a functional block diagram of one illustrative configuration of the IMD 16. As shown, the IMD 16 may include a control module 81, a therapy delivery module 84 (e.g., which may include a stimulation generator), a sensing module 86, and a power source 90.

The control module, or apparatus, 81 may include a processor 80, memory 82, and a telemetry module, or apparatus, 88. The memory 82 may include computer-readable instructions that, when executed, e.g., by the processor 80, cause the IMD 16 and/or the control module 81 to perform various functions attributed to the IMD 16 and/or the control module 81 described herein. Further, the memory 82 may include any volatile, non-volatile, magnetic, optical, and/or electrical media, such as a randomaccess memory (RAM), read-only memory (ROM), non-volatile RAM (NVRAM), electrically-erasable programmable ROM (EEPROM), flash memory, and/or any other digital media. An illustrative capture management module may be the left ventricular capture management (LVCM) module described in U.S. Pat. No. 7,684,863 entitled "LV THRESHOLD MEASUREMENT AND CAPTURE MANAGEMENT" and issued March 23, 2010.

The processor 80 of the control module 81 may include any one or more of a microprocessor, a controller, a digital signal processor (DSP), an application specific integrated circuit (ASIC), a field-programmable gate array (FPGA), and/or equivalent discrete or integrated logic circuitry. In some examples, the processor 80 may include multiple components, such as any combination of one or more microprocessors, one or more controllers, one or more DSPs, one or more ASICs, and/or one or more FPGAs, as well as other discrete or integrated logic circuitry. The functions attributed to the processor 80 herein may be embodied as software, firmware, hardware, or any combination thereof.

The control module 81 may control the therapy delivery module 84 to deliver therapy (e.g., electrical stimulation therapy such as pacing) to the heart 12 according to a selected one or more therapy programs, which may be stored in the memory 82. More, specifically, the control module 81 (e.g., the processor 80) may control various parameters of the electrical stimulus delivered by the therapy delivery module 84 such as, e.g., A-V delays, V-V delays, pacing pulses with the amplitudes, pulse widths, frequency, or electrode polarities, etc., which may be specified by one or more selected therapy programs (e.g., A-V and/or V-V delay adjustment programs, pacing therapy programs, pacing recovery programs, capture management programs, etc.). As shown, the therapy delivery module 84 is electrically coupled to electrodes 40, 42, 44, 45, 46, 47, 48, 50, 58, 62, 64, 66, e.g., via conductors of the respective lead 18, 20, 22, or, in the case of housing electrode 58, via an electrical conductor disposed within housing 60 of IMD 16. Therapy delivery module 84 may be configured to generate and deliver electrical stimulation therapy such as pacing therapy to the heart 12 using one or more of the electrodes 40, 42, 44, 45, 46, 47, 48, 50, 58, 62, 64, 66.

For example, therapy delivery module 84 may deliver pacing stimulus (e.g., pacing pulses) via ring electrodes 40, 44, 45, 46, 47, 48 coupled to leads 18, 20, 22 and/or helical tip electrodes 42, 50 of leads 18, 22. Further, for example, therapy delivery module 84 may deliver defibrillation shocks to heart 12 via at least two of electrodes 58, 62, 64, 66. In some examples, therapy delivery module 84 may be configured to deliver pacing, cardioversion, or defibrillation stimulation in the form of electrical pulses. In other examples, therapy delivery module 84 may be configured deliver one or more of these types of stimulation in the form of other signals, such as sine waves, square waves, and/or other substantially continuous time signals.

The IMD 16 may further include a switch module 85 and the control module 81 (e.g., the processor 80) may use the switch module 85 to select, e.g., via a data/address bus, which of the available electrodes are used to deliver therapy such as pacing pulses for pacing therapy, or which of the available electrodes are used for sensing. The switch module 85 may include a switch array, switch matrix, multiplexer, or any other type of switching device suitable to selectively couple the sensing module 86 and/or the therapy delivery module 84 to one or more selected electrodes. More specifically, the therapy delivery module 84 may include a plurality of pacing output circuits. Each pacing output circuit of the plurality of pacing output circuits may be selectively coupled, e.g., using the switch module 85, to one or more of the electrodes 40, 42, 44, 45, 46, 47, 48, 50, 58, 62, 64, 66 (e.g., a pair of electrodes for delivery of therapy to a bipolar or multipolar pacing vector). In other words, each electrode can be selectively coupled to one of the pacing output circuits of the therapy delivery module using the switching module 85.

The sensing module 86 is coupled (e.g., electrically coupled) to sensing apparatus, which may include, among additional sensing apparatus, the electrodes 40, 42, 44, 45, 46, 47, 48, 50, 58, 62, 64, 66 to monitor electrical activity of the heart 12, e.g., electrocardiogram (ECG)/electrogram (EGM) signals, etc. The ECG/EGM signals may be used to measure or monitor activation times (e.g., ventricular activations times, etc.), heart rate (HR), heart rate variability (HRV), heart rate turbulence (HRT), deceleration/acceleration capacity, deceleration sequence incidence, T-wave alternans (TWA), P-wave to P-wave intervals (also referred to as the P-P intervals or A-A intervals), R-wave to R-wave intervals (also referred to as the R-R intervals or V-V intervals), P-wave to QRS complex intervals (also referred to as the P-R intervals, A-V intervals, or P-Q intervals), QRS-complex morphology, ST segment (i.e., the segment that connects the QRS complex and the T-wave), T-wave changes, QT intervals, electrical vectors, etc.

The switch module 85 may also be used with the sensing module 86 to select which of the available electrodes are used, or enabled, to, e.g., sense electrical activity of the patient's heart (e.g., one or more electrical vectors of the patient's heart using any combination of the electrodes 40, 42, 44, 45, 46, 47, 48, 50, 58, 62, 64, 66). Likewise, the switch module 85 may also be used with the sensing module 86 to select which of the available electrodes are not to be used (e.g., disabled) to, e.g., sense electrical activity of the patient's heart (e.g., one or more electrical vectors of the patient's heart using any combination of the electrodes 40, 42, 44, 45, 46, 47, 48, 50, 58, 62, 64, 66), etc. In some examples, the control module 81 may select the electrodes that function as sensing electrodes via the switch module within the sensing module 86, e.g., by providing signals via a data/address bus.

In some examples, sensing module 86 includes a channel that includes an amplifier with a relatively wider pass band than the R-wave or P-wave amplifiers. Signals from the selected sensing electrodes may be provided to a multiplexer, and thereafter converted to multi-bit digital signals by an analog-to-digital converter for storage in memory 82, e.g., as an electrogram (EGM). In some examples, the storage of such EGMs in memory 82 may be under the control of a direct memory access circuit.

In some examples, the control module 81 may operate as an interruptdriven device and may be responsive to interrupts from pacer timing and control module, where the interrupts may correspond to the occurrences of sensed P-waves and R-waves and the generation of cardiac pacing pulses. Any necessary mathematical calculations may be performed by the processor 80 and any updating of the values or intervals controlled by the pacer timing and control module may take place following such interrupts. A portion of memory 82 may be configured as a plurality of recirculating buffers, capable of holding one or more series of measured intervals, which may be analyzed by, e.g., the processor 80 in response to the occurrence of a pace or sense interrupt to determine whether the patient's heart 12 is presently exhibiting atrial or ventricular tachyarrhythmia.

The telemetry module 88 of the control module 81 may include any suitable hardware, firmware, software, or any combination thereof for communicating with another device, such as a programmer. For example, under the control of the processor 80, the telemetry module 88 may receive downlink telemetry from and send uplink telemetry to a programmer with the aid of an antenna, which may be internal and/or external. The processor 80 may provide the data to be uplinked to a programmer and the control signals for the telemetry circuit within the telemetry module 88, e.g., via an address/data bus. In some examples, the telemetry module 88 may provide received data to the processor 80 via a multiplexer.

The various components of the IMD 16 are further coupled to a power source 90, which may include a rechargeable or non-rechargeable battery. A non-rechargeable battery may be selected to last for several years, while a rechargeable battery may be inductively charged from an external device, e.g., on a daily or weekly basis.

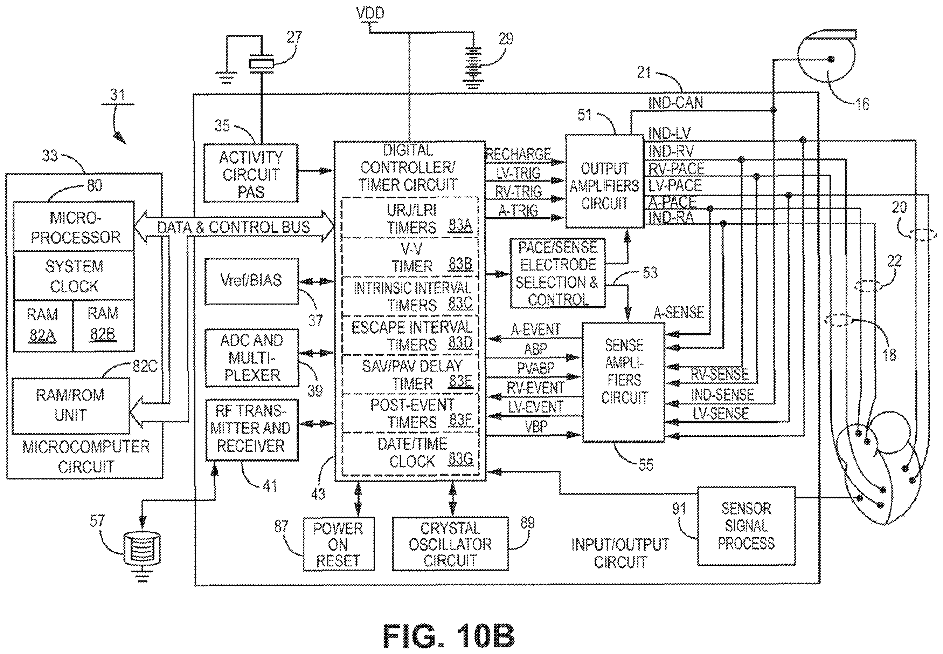

FIG. 10B is another embodiment of a functional block diagram for IMD 16 that depicts bipolar RA lead 22, bipolar RV lead 18, and bipolar LV CS lead 20 without the LA CS pace/sense electrodes and coupled with an implantable pulse generator (IPG) circuit 31 having programmable modes and parameters of a bi-ventricular DDD/R type known in the pacing art. In turn, the sensor signal processing circuit 91 indirectly couples to the timing circuit 43 and via data and control bus to microcomputer circuitry 33. The IPG circuit 31 is illustrated in a functional block diagram divided generally into a microcomputer circuit 33 and a pacing circuit 21. The pacing circuit 21 includes the digital controller/timer circuit 43, the output amplifiers circuit 51, the sense amplifiers circuit 55, the RF telemetry transceiver 41, the activity sensor circuit 35 as well as a number of other circuits and components described below.

Crystal oscillator circuit 89 provides the basic timing clock for the pacing circuit 21 while battery 29 provides power. Power-on-reset circuit 87 responds to initial connection of the circuit to the battery for defining an initial operating condition and similarly, resets the operative state of the device in response to detection of a low battery condition. Reference mode circuit 37 generates stable voltage reference and currents for the analog circuits within the pacing circuit 21. Analog-to-digital converter (ADC) and multiplexer circuit 39 digitize analog signals and voltage to provide, e.g., real time telemetry of cardiac signals from sense amplifiers 55 for uplink transmission via RF transmitter and receiver circuit 41. Voltage reference and bias circuit 37, ADC and multiplexer 39, power-on-reset circuit 87, and crystal oscillator circuit 89 may correspond to any of those used in illustrative implantable cardiac pacemakers.

If the IPG is programmed to a rate responsive mode, the signals output by one or more physiologic sensors are employed as a rate control parameter (RCP) to derive a physiologic escape interval. For example, the escape interval is adjusted proportionally to the patient's activity level developed in the patient activity sensor (PAS) circuit 35 in the depicted, illustrative IPG circuit 31. The patient activity sensor 27 is coupled to the IPG housing and may take the form of a piezoelectric crystal transducer. The output signal of the patient activity sensor 27 may be processed and used as an RCP. Sensor 27 generates electrical signals in response to sensed physical activity that are processed by activity circuit 35 and provided to digital controller/timer circuit 43. Activity circuit 35 and associated sensor 27 may correspond to the circuitry disclosed in U.S. Pat. No. 5,052,388 entitled "METHOD AND APPARATUS FOR IMPLEMENTING ACTIVITY SENSING IN A PULSE GENERATOR" and issued on October 1, 1991 and U.S. Pat. No. 4,428,378 entitled "RATE ADAPTIVE PACER" and issued on January 31, 1984. Similarly, the illustrative systems, apparatus, and methods described herein may be practiced in conjunction with alternate types of sensors such as oxygenation sensors, pressure sensors, pH sensors, and respiration sensors, for use in providing rate responsive pacing capabilities. Alternately, QT time may be used as a rate indicating parameter, in which case no extra sensor is required. Similarly, the illustrative embodiments described herein may also be practiced in non-rate responsive pacemakers.

Data transmission to and from the external programmer is accomplished by way of the telemetry antenna 57 and an associated RF transceiver 41, which serves both to demodulate received downlink telemetry and to transmit uplink telemetry. Uplink telemetry capabilities may include the ability to transmit stored digital information, e.g., operating modes and parameters, EGM histograms, and other events, as well as real time EGMs of atrial and/or ventricular electrical activity and marker channel pulses indicating the occurrence of sensed and paced depolarizations in the atrium and ventricle.

Microcomputer 33 contains a microprocessor 80 and associated system clock and on-processor RAM and ROM chips 82A and 82B, respectively. In addition, microcomputer circuit 33 includes a separate RAM/ROM chip 82C to provide additional memory capacity. Microprocessor 80 normally operates in a reduced power consumption mode and is interrupt driven. Microprocessor 80 is awakened in response to defined interrupt events, which may include A-TRIG, RV-TRIG, LV-TRIG signals generated by timers in digital timer/controller circuit 43 and A-EVENT, RV-EVENT, and LV-EVENT signals generated by sense amplifiers circuit 55, among others. The specific values of the intervals and delays timed out by digital controller/timer circuit 43 are controlled by the microcomputer circuit 33 by way of data and control bus from programmed-in parameter values and operating modes. In addition, if programmed to operate as a rate responsive pacemaker, a timed interrupt, e.g., every cycle or every two seconds, may be provided in order to allow the microprocessor to analyze the activity sensor data and update the basic A-A, V-A, or V-V escape interval, as applicable. In addition, the microprocessor 80 may also serve to define variable, operative A-V delay intervals, V-V delay intervals, and the energy delivered to each ventricle and/or atrium.

In one embodiment, microprocessor 80 is a custom microprocessor adapted to fetch and execute instructions stored in RAM/ROM unit 82 in a conventional manner. It is contemplated, however, that other implementations may be suitable to practice the present disclosure. For example, an off-the-shelf, commercially available microprocessor or microcontroller, or custom application-specific, hardwired logic, or state-machine type circuit may perform the functions of microprocessor 80.

Digital controller/timer circuit 43 operates under the general control of the microcomputer 33 to control timing and other functions within the pacing circuit 21 and includes a set of timing and associated logic circuits of which certain ones pertinent to the present disclosure are depicted. The depicted timing circuits include URI/LRI timers 83A, V-V delay timer 83B, intrinsic interval timers 83C for timing elapsed V-EVENT to V-EVENT intervals or V-EVENT to A-EVENT intervals or the V-V conduction interval, escape interval timers 83D for timing A-A, V-A, and/or V-V pacing escape intervals, an A-V delay interval timer 83E for timing the A-LVp delay (or A-RVp delay) from a preceding A-EVENT or A-TRIG, a post-ventricular timer 83F for timing post-ventricular time periods, and a date/time clock 83G.

The A-V delay interval timer 83E is loaded with an appropriate delay interval for one ventricular chamber (e.g., either an A-RVp delay or an A-LVp) to time-out starting from a preceding A-PACE or A-EVENT. The interval timer 83E triggers pacing stimulus delivery and can be based on one or more prior cardiac cycles (or from a data set empirically derived for a given patient).

The post-event timer 83F times out the post-ventricular time period following an RV-EVENT or LV-EVENT or a RV-TRIG or LV-TRIG and post-atrial time periods following an A-EVENT or A-TRIG. The durations of the post-event time periods may also be selected as programmable parameters stored in the microcomputer 33. The post-ventricular time periods include the PVARP, a post-atrial ventricular blanking period (PAVBP), a ventricular blanking period (VBP), a post-ventricular atrial blanking period (PVARP) and a ventricular refractory period (VRP) although other periods can be suitably defined depending, at least in part, on the operative circuitry employed in the pacing engine. The post-atrial time periods include an atrial refractory period (ARP) during which an A-EVENT is ignored for the purpose of resetting any A-V delay, and an atrial blanking period (ABP) during which atrial sensing is disabled. It should be noted that the starting of the post-atrial time periods and the A-V delays can be commenced substantially simultaneously with the start or end of each A-EVENT or A-TRIG or, in the latter case, upon the end of the A-PACE which may follow the A-TRIG. Similarly, the starting of the post-ventricular time periods and the V-A escape interval can be commenced substantially simultaneously with the start or end of the V-EVENT or V-TRIG or, in the latter case, upon the end of the V-PACE which may follow the V-TRIG. The microprocessor 80 also optionally calculates A-V delays, V-V delays, post-ventricular time periods, and post-atrial time periods that vary with the sensor-based escape interval established in response to the RCP(s) and/or with the intrinsic atrial and/or ventricular rate.

The output amplifiers circuit 51 contains a RA pace pulse generator (and a LA pace pulse generator if LA pacing is provided), a RV pace pulse generator, a LV pace pulse generator, and/or any other pulse generator configured to provide atrial and ventricular pacing. In order to trigger generation of an RV-PACE or LV-PACE pulse, digital controller/timer circuit 43 generates the RV-TRIG signal at the time-out of the A-RVp delay (in the case of RV pre-excitation) or the LV-TRIG at the time-out of the A-LVp delay (in the case of LV pre-excitation) provided by A-V delay interval timer 83E (or the V-V delay timer 83B). Similarly, digital controller/timer circuit 43 generates an RA-TRIG signal that triggers output of an RA-PACE pulse (or an LA-TRIG signal that triggers output of an LA-PACE pulse, if provided) at the end of the V-A escape interval timed by escape interval timers 83D.

The output amplifiers circuit 51 includes switching circuits for coupling selected pace electrode pairs from among the lead conductors and the IND-CAN electrode 20 to the RA pace pulse generator (and LA pace pulse generator if provided), RV pace pulse generator and LV pace pulse generator. Pace/sense electrode pair selection and control circuit 53 selects lead conductors and associated pace electrode pairs to be coupled with the atrial and ventricular output amplifiers within output amplifiers circuit 51 for accomplishing RA, LA, RV and LV pacing.

The sense amplifiers circuit 55 contains sense amplifiers for atrial and ventricular pacing and sensing. High impedance P-wave and R-wave sense amplifiers may be used to amplify a voltage difference signal that is generated across the sense electrode pairs by the passage of cardiac depolarization wavefronts. The high impedance sense amplifiers use high gain to amplify the low amplitude signals and rely on pass band filters, time domain filtering and amplitude threshold comparison to discriminate a P-wave or R-wave from background electrical noise. Digital controller/timer circuit 43 controls sensitivity settings of the atrial and ventricular sense amplifiers 55.

The sense amplifiers may be uncoupled from the sense electrodes during the blanking periods before, during, and after delivery of a pace pulse to any of the pace electrodes of the pacing system to avoid saturation of the sense amplifiers. The sense amplifiers circuit 55 includes blanking circuits for uncoupling the selected pairs of the lead conductors and the IND-CAN electrode 20 from the inputs of the RA sense amplifier (and LA sense amplifier if provided), RV sense amplifier and LV sense amplifier during the ABP, PVABP and VBP. The sense amplifiers circuit 55 also includes switching circuits for coupling selected sense electrode lead conductors and the IND-CAN electrode 20 to the RA sense amplifier (and LA sense amplifier if provided), RV sense amplifier and LV sense amplifier. Again, sense electrode selection and control circuit 53 selects conductors and associated sense electrode pairs to be coupled with the atrial and ventricular sense amplifiers within the output amplifiers circuit 51 and sense amplifiers circuit 55 for accomplishing RA, LA, RV, and LV sensing along desired unipolar and bipolar sensing vectors.

Right atrial depolarizations or P-waves in the RA-SENSE signal that are sensed by the RA sense amplifier result in a RA-EVENT signal that is communicated to the digital controller/timer circuit 43. Similarly, left atrial depolarizations or P-waves in the LA-SENSE signal that are sensed by the LA sense amplifier, if provided, result in a LA-EVENT signal that is communicated to the digital controller/timer circuit 43. Ventricular depolarizations or R-waves in the RV-SENSE signal are sensed by a ventricular sense amplifier result in an RV-EVENT signal that is communicated to the digital controller/timer circuit 43. Similarly, ventricular depolarizations or R-waves in the LV-SENSE signal are sensed by a ventricular sense amplifier result in an LV-EVENT signal that is communicated to the digital controller/timer circuit 43. The RV-EVENT, LV-EVENT, and RA-EVENT, LA-SENSE signals may be refractory or nonrefractory and can inadvertently be triggered by electrical noise signals or aberrantly conducted depolarization waves rather than true R-waves or P-waves.

The techniques described in this disclosure, including those attributed to the IMD 16, the remote computing apparatus 140, and/or various constituent components, may be implemented, at least in part, in hardware, software, firmware, or any combination thereof. For example, various aspects of the techniques may be implemented within one or more processors, including one or more microprocessors, DSPs, ASICs, FPGAs, or any other equivalent integrated or discrete logic circuitry, as well as any combinations of such components, embodied in programmers, such as physician or patient programmers, stimulators, image processing devices, or other devices. The term "module," "processor," or "processing circuitry" may generally refer to any of the foregoing logic circuitry, alone or in combination with other logic circuitry, or any other equivalent circuitry.

Such hardware, software, and/or firmware may be implemented within the same device or within separate devices to support the various operations and functions described in this disclosure. In addition, any of the described units, modules, or components may be implemented together or separately as discrete but interoperable logic devices. Depiction of different features as modules or units is intended to highlight different functional aspects and does not necessarily imply that such modules or units must be realized by separate hardware or software components. Rather, functionality associated with one or more modules or units may be performed by separate hardware or software components or integrated within common or separate hardware or software components.

When implemented in software, the functionality ascribed to the systems, devices and techniques described in this disclosure may be embodied as instructions on a computer-readable medium such as RAM, ROM, NVRAM, EEPROM, FLASH memory, magnetic data storage media, optical data storage media, or the like. The instructions may be executed by processing circuitry and/or one or more processors to support one or more aspects of the functionality described in this disclosure.

This disclosure has been provided with reference to illustrative examples and embodiments and is not meant to be construed in a limiting sense. As described previously, one skilled in the art will recognize that other various illustrative applications may use the techniques as described herein to take advantage of the beneficial characteristics of the apparatus and methods described herein. Various modifications of the illustrative examples and embodiments will be apparent upon
reference to this description.

## Claims

1. A system (100) comprising:
electrode apparatus (110) comprising a plurality of electrodes (112) to monitor electrical activity from tissue of a patient (14); and
computing apparatus (140) comprising processing circuitry and coupled to the electrode apparatus, the computing apparatus configured to:
monitor electrical activity using the plurality of electrodes to generate a plurality of cardiac signals over an analysis time period,
generate a dispersion signal from the plurality of cardiac signals, wherein the dispersion signal is representative of the dispersion of the plurality of cardiac signals over the analysis time period,
select a low dispersion time period (260) within the analysis time period based on rate of change of the dispersion signal, and
determine whether each of the plurality of cardiac signals is stable based on the cardiac signal within the low dispersion time period.

2. The system of claim 1, wherein generating a dispersion signal from the plurality of cardiac signals comprises determining a standard deviation of the plurality of cardiac signals over the analysis time period.

3. The system of any of claims 1-2, wherein selecting the low dispersion time period within the analysis time period based on rate of change of the dispersion signal comprises:
determining a minimum rate of change of the dispersion signal over a sliding window within the analysis time period; and
identifying the low dispersion time period based on the determined minimum rate of change of the dispersion signal over the sliding window.

4. The system of claim 3, wherein the low dispersion time period and the sliding window are less than or equal to 200 milliseconds.

5. The system of any of claims 1-4, wherein selecting the low dispersion time period within the analysis time period based on rate of change of the dispersion signal comprises:
determining a first minimum rate of change of the dispersion signal over a first sliding window within the analysis time period;
identifying an initial low dispersion time period (260) based on the determined first minimum rate of change of the dispersion signal over the first sliding window within the analysis time period;
determining a second minimum rate of change of the dispersion signal over a second sliding window within the initial low dispersion time period; and
identifying the low dispersion time period (262) based on the determined second minimum rate of change of the dispersion signal over the second sliding window within the initial low dispersion time period.

6. The system of claim 5, wherein the second sliding window is less than the first sliding window.

7. The system of any of claims 1-6, wherein determining whether each of the plurality of cardiac signals is stable based on the cardiac signal within the low dispersion time period comprises
generating a peak-to-peak amplitude for each of the plurality of cardiac signals within the low dispersion time period; and
determining whether each of the plurality of cardiac signals is stable if the peak-to-peak amplitude of the cardiac signal within the low dispersion time period is less than or equal to a stability threshold.

8. The system of claim 7, wherein the stability threshold is 2.5 times the median peak-to-peak amplitude of the plurality of cardiac signals within the low dispersion time period.

9. The system of any of claims 1-8, wherein the computing apparatus is further configured to removing low amplitude signals from the plurality of cardiac signals prior to generating the dispersion signal.

10. The system of claim 9, wherein removing low amplitude signals from the plurality of cardiac signals prior to generating the dispersion signal comprises:
generating a peak-to-peak amplitude for each of the plurality of cardiac signals within the analysis time period; and
determining that each of the plurality of cardiac signals is low amplitude if the peak-to-peak amplitude of the cardiac signal within the analysis time period is less than or equal to a low amplitude threshold.

11. The system of any of claims 1-10, wherein the computing apparatus is further configured to determine a QRS onset and a QRS offset within the analysis time period based on the stable cardiac signals of the plurality of cardiac signals.

12. The system of any of claims 1-11, wherein the plurality of electrodes comprises a plurality of external electrodes to be located proximate the patient's skin.

## Patentansprüche

1. System (100), umfassend:
eine Elektrodeneinrichtung (110), umfassend eine Vielzahl von Elektroden (112), um elektrische Aktivität von Gewebe eines Patienten (14) zu überwachen; und
eine Recheneinrichtung (140), umfassend eine Verarbeitungsschaltung und die an die Elektrodeneinrichtung gekoppelt ist, wobei die Recheneinrichtung konfiguriert ist zum:
Überwachen elektrischer Aktivität unter Verwendung der Vielzahl von Elektroden, um im Laufe eines Analysezeitraums eine Vielzahl von Herzsignalen zu erzeugen,
Erzeugen eines Dispersionssignals aus der Vielzahl von Herzsignalen, wobei das Dispersionssignal für die Dispersion der Vielzahl von Herzsignalen im Laufe des Analysezeitraums darstellend ist,
Auswählen eines Zeitraums (260) geringer Dispersion innerhalb des Analysezeitraums basierend auf einer Änderungsrate des Dispersionssignals, und
Bestimmen, ob jedes der Vielzahl von Herzsignalen stabil ist, basierend auf dem Herzsignal innerhalb des Zeitraums geringer Dispersion.

2. System nach Anspruch 1, wobei das Erzeugen eines Dispersionssignals aus der Vielzahl von Herzsignalen das Bestimmen einer Standardabweichung der Vielzahl von Herzsignalen im Laufe des Analysezeitraums umfasst.

3. System nach einem der Ansprüche 1 bis 2, wobei das Auswählen des Zeitraums geringer Dispersion innerhalb des Analysezeitraums basierend auf der Änderungsrate des Dispersionssignals umfasst:
Bestimmen einer minimalen Änderungsrate des Dispersionssignals im Laufe eines gleitenden Fensters innerhalb des Analysezeitraums; und
Identifizieren des Zeitraums geringer Dispersion basierend auf der bestimmten minimalen Änderungsrate des Dispersionssignals im Laufe des gleitenden Fensters.

4. System nach Anspruch 3, wobei der Zeitraum geringer Dispersion und das gleitende Fenster kleiner als oder gleich 200 Millisekunden sind.

5. System nach einem der Ansprüche 1 bis 4, wobei das Auswählen des Zeitraums geringer Dispersion innerhalb des Analysezeitraums basierend auf der Änderungsrate des Dispersionssignals umfasst: Bestimmen einer ersten minimalen Änderungsrate des Dispersionssignals im Laufe eines ersten gleitenden Fensters innerhalb des Analysezeitraums;
Identifizieren eines anfänglichen Zeitraums (260) geringer Dispersion basierend auf der bestimmten ersten minimalen Änderungsrate des Dispersionssignals im Laufe des ersten gleitenden Fensters innerhalb des Analysezeitraums;
Bestimmen einer zweiten minimalen Änderungsrate des Dispersionssignals im Laufe eines zweiten gleitenden Fensters innerhalb des anfänglichen Zeitraums geringer Dispersion; und
Identifizieren des Zeitraums (262) geringer Dispersion basierend auf der bestimmten zweiten minimalen Änderungsrate des Dispersionssignals im Laufe des zweiten gleitenden Fensters innerhalb des anfänglichen Zeitraums geringer Dispersion.

6. System nach Anspruch 5, wobei das zweite gleitende Fenster kleiner als das erste gleitende Fenster ist.

7. System nach einem der Ansprüche 1 bis 6, wobei das Bestimmen, ob jedes der Vielzahl von Herzsignalen stabil ist, basierend auf dem Herzsignal innerhalb des Zeitraums geringer Dispersion umfasst:
Erzeugen einer Spitze-zu-Spitze-Amplitude für jedes der Vielzahl von Herzsignalen innerhalb des Zeitraums geringer Dispersion; und
Bestimmen, ob jedes der Vielzahl von Herzsignalen stabil ist, falls die Spitze-zu-Spitze-Amplitude des Herzsignals innerhalb des Zeitraums geringer Dispersion kleiner als oder gleich einem Stabilitätsschwellenwert ist.

8. System nach Anspruch 7, wobei der Stabilitätsschwellenwert das 2,5-Fache der medianen Spitze-zu-Spitze-Amplitude der Vielzahl von Herzsignalen innerhalb des Zeitraums geringer Dispersion beträgt.

9. System nach einem der Ansprüche 1 bis 8, wobei die Recheneinrichtung ferner konfiguriert ist, um Signale geringer Amplitude aus der Vielzahl von Herzsignalen vor dem Erzeugen des Dispersionssignals zu entfernen.

10. System nach Anspruch 9, wobei das Entfernen von Signalen geringer Amplitude aus der Vielzahl von Herzsignalen vor dem Erzeugen des Dispersionssignals umfasst:
Erzeugen einer Spitze-zu-Spitze-Amplitude für jedes der Vielzahl von Herzsignalen innerhalb des Analysezeitraums; und
Bestimmen, dass jedes der Vielzahl von Herzsignalen von geringer Amplitude ist, falls die Spitze-zu-Spitze-Amplitude des Herzsignals innerhalb des Analysezeitraums kleiner als oder gleich einem Schwellenwert geringer Amplitude ist.

11. System nach einem der Ansprüche 1 bis 10, wobei die Recheneinrichtung ferner konfiguriert ist, um einen QRS-Beginn und ein QRS-Ende innerhalb des Analysezeitraums basierend auf den stabilen Herzsignalen der Vielzahl von Herzsignalen zu bestimmen.

12. System nach einem der Ansprüche 1 bis 11, wobei die Vielzahl von Elektroden eine Vielzahl von externen Elektroden umfasst, die in der Nähe der Haut des Patienten zu platzieren sind.

## Revendications

1. Système (100) comprenant :
un appareil à électrodes (110) comprenant une pluralité d'électrodes (112) pour surveiller une activité électrique d'un tissu d'un patient (14) ; et
un appareil informatique (140) comprenant un circuit de traitement et couplé à l'appareil à électrodes, l'appareil informatique étant configuré pour :
surveiller l'activité électrique à l'aide de la pluralité d'électrodes afin de générer une pluralité de signaux cardiaques sur un laps de temps d'analyse,
générer un signal de dispersion à partir de la pluralité de signaux cardiaques, dans lequel le signal de dispersion est représentatif de la dispersion de la pluralité de signaux cardiaques sur le laps de temps d'analyse,
sélectionner un laps de temps de faible dispersion (260) dans le laps de temps d'analyse en fonction du taux de changement du signal de dispersion, et
déterminer si chacun de la pluralité de signaux cardiaques est stable en fonction du signal cardiaque dans le laps de temps de faible dispersion.

2. Système selon la revendication 1, dans lequel la génération d'un signal de dispersion à partir de la pluralité de signaux cardiaques comprend la détermination d'une déviation standard de la pluralité de signaux cardiaques sur le laps de temps d'analyse.

3. Système selon l'une quelconque des revendications 1-2, dans lequel la sélection du laps de temps de faible dispersion dans le laps de temps d'analyse en fonction du taux de changement du signal de dispersion comprend :
la détermination d'un taux minimal de changement du signal de dispersion sur une fenêtre glissante dans le laps de temps d'analyse ; et
l'identification du laps de temps de faible dispersion en fonction du taux minimal de changement déterminé du signal de dispersion sur la fenêtre glissante.

4. Système selon la revendication 3, dans lequel le laps de temps de faible dispersion et la fenêtre glissante sont inférieurs ou égaux à 200 millisecondes.

5. Système selon l'une quelconque des revendications 1-4, dans lequel la sélection du laps de temps de faible dispersion dans le laps de temps d'analyse en fonction du taux de changement du signal de dispersion comprend : la détermination d'un premier taux minimal de changement du signal de dispersion sur une première fenêtre glissante dans le laps de temps d'analyse ;
l'identification d'un laps de temps initial de faible dispersion (260) en fonction du premier taux minimal de changement déterminé du signal de dispersion sur la première fenêtre glissante dans le laps de temps d'analyse ;
la détermination d'un second taux minimal de changement du signal de dispersion sur une seconde fenêtre glissante dans le laps de temps initial de faible dispersion ; et
l'identification du laps de temps de faible dispersion (262) en fonction du second taux minimal de changement déterminé du signal de dispersion sur la seconde fenêtre glissante dans le laps de temps initial de faible dispersion.

6. Système selon la revendication 5, dans lequel la seconde fenêtre coulissante est inférieure à la première fenêtre glissante.

7. Système selon l'une quelconque des revendications 1-6, dans lequel la détermination si chacun de la pluralité de signaux cardiaques est stable en fonction du signal cardiaque dans le laps de temps de faible dispersion comprend
la génération d'une amplitude crête à crête pour chacun de la pluralité de signaux cardiaques dans le laps de temps de faible dispersion ; et
la détermination si chacun de la pluralité de signaux cardiaques est stable si l'amplitude crête à crête du signal cardiaque dans le laps de temps de faible dispersion est inférieure ou égale à un seuil de stabilité.

8. Système selon la revendication 7, dans lequel le seuil de stabilité est 2,5 fois l'amplitude médiane crête à crête de la pluralité de signaux cardiaques dans le laps de temps de faible dispersion.

9. Système selon l'une quelconque des revendications 1-8, dans lequel l'appareil informatique est en outre configuré pour retirer les signaux de faible amplitude de la pluralité de signaux cardiaques avant la génération du signal de dispersion.

10. Système selon la revendication 9, dans lequel le retrait des signaux de faible amplitude de la pluralité de signaux cardiaques avant la génération du signal de dispersion comprend :
la génération d'une amplitude crête à crête pour chacun de la pluralité de signaux cardiaques dans le laps de temps d'analyse ; et
la détermination que chacun de la pluralité de signaux cardiaques est de faible amplitude si l'amplitude crête à crête du signal cardiaque dans le laps de temps d'analyse est inférieure ou égale à un seuil de faible amplitude.

11. Système selon l'une quelconque des revendications 1-10, dans lequel l'appareil informatique est en outre configuré pour déterminer un début du complexe QRS et une fin du complexe QRS dans le laps de temps d'analyse en fonction des signaux cardiaques stables de la pluralité de signaux cardiaques.

12. Système selon l'une quelconque des revendications 1-11, dans lequel la pluralité d'électrodes comprend une pluralité d'électrodes externes destinées à être placées à proximité de la peau du patient.
